# EUROPÄISCHE PATENTSCHRIFT

(11) **EP 2 616 704 B1**
(45) Veröffentlichungstag und Bekanntmachung des Hinweises auf die Patenterteilung: **12.11.2014**
(21) Anmeldenummer: 11770690.3
(22) Anmeldetag: 15.09.2011
(51) Int. Cl.: F16D 37/02, F16D 57/00, F16C 33/66

(54) **MAGNETORHEOLOGISCHE ÜBERTRAGUNGSVORRICHTUNG**
MAGNETORHEOLOGICAL TRANSMISSION DEVICE
DISPOSITIF DE TRANSMISSION MAGNÉTORHÉOLOGIQUE

(30) Priorität: 23.12.2010 DE 102010055833; 15.09.2010 DE 102010045436
(43) Veröffentlichungstag der Anmeldung: 24.07.2013
(73) Patentinhaber: Inventus Engineering GmbH, 6771 St. Anton i. M. (AT)
(72) Erfinder: BATTLOGG, Stefan, A-6771 St. Anton I.M. (AT)
(74) Vertreter: BSB Intellectual Property Law
(86) Internationale Anmeldenummer: PCT/EP2011/004623
(87) Internationale Veröffentlichungsnummer: WO 2012/034697

(56) Entgegenhaltungen:
- DE-A1-102006 034 966
- DE-A1-102007 028 990
- DE-B3-102004 009 906
- JP-A- 10 176 719

## Beschreibung

Die vorliegende Erfindung betrifft eine magnetorheologische Übertragungsvorrichtung und insbesondere eine magnetorheologische Kraft- oder Drehmomentübertragungseinrichtung, wobei die Übertragung zwischen einer ersten Komponente und mindestens einer sich relativ zu dieser bewegenden oder ruhenden zweiten Komponente durch die magnetorheologischen Eigenschaften einer sich zwischen den Komponenten befindenden Flüssigkeit verändert werden kann. Beispielsweise kann die vorliegende Erfindung das Drehmoment einer Antriebsachse zu einer Abtriebsachse hin wahlweise verringern.

Die erfindungsgemäße magnetorheologische Übertragungsvorrichtung kann auf vielfältigen technischen Gebieten eingesetzt werden, so z.B. an Fahrzeugen oder industriellen Anlagen als Kupplung oder Bremse oder zur Herstellung variabler Anschläge einer Fahrzeugtür. Die Erfindung kann aber auch z.B. als Lenkradsperre an einer Lenksäule von Automobilen oder sonstigen Zweirädern eingesetzt werden oder auch als Antischlupfregelung, Drehmomentverteiler, Lüfterkupplung usw. bei Fahrzeugen. Möglich ist der Einsatz auch als Gelenk an Prothesen, künstlichen Gliedmaßen oder auf sonstigen technischen Gebieten.

Im Stand der Technik sind verschiedenste Kupplungen und dergleichen bekannt geworden, bei denen beispielsweise eine zweite Komponente über die Aktivierung der Kupplung in synchrone Drehbewegung mit einer ersten Komponente gebracht wird. Dazu können beispielsweise Kupplungsscheiben, die mit einem Reibbelag oder dergleichen versehen sind, einander kontaktieren, um durch den zunächst schleifenden Kontakt die zweite Komponente auf die Drehzahl der ersten Komponente zu bringen.

Neben herkömmlichen Kupplungen und Bremsen mit konventionellen Reibbelägen sind auch Kupplungen bekannt geworden, bei denen zwischen zwei als Kupplungsscheiben dienenden Komponenten beispielsweise ein magnetorheologisches Fluid vorgesehen ist. Magnetorheologische Fluide weisen beispielsweise in einem Öl verteilt feinste ferromagnetische Partikel wie beispielsweise Carbonyleisenpulver auf. In magnetorheologischen Flüssigkeiten werden kugelförmige Partikel mit einem herstellungsbedingten Durchmesser von 1 bis 10 Mikrometer verwendet, wobei die Partikelgröße nicht einheitlich ist. Wird ein solches magnetorheologisches Fluid mit einem Magnetfeld beaufschlagt, so verketten sich die Carbonyleisenpartikel des magnetorheologischen Fluides entlang der Magnetfeldlinien, sodass die rheologischen Eigenschaften des magnetorheologischen Fluides (MRF) abhängig von Form und Stärke des Magnetfeldes erheblich beeinflusst werden.

Mit der DE 10 2004 009 906 B3 ist ein Wälzlager bekannt geworden, mit dem eine Lenksäule drehbar gelagert wird. Alleine durch die Erhöhung der Viskosität soll das gesetzlich vorgeschriebene Mindestdrehmoment von über 100 Nm erreicht werden, mit dem eine Lenksäule im abgesperrten Zustand mindestens blockiert werden muss. Ein solches Lager ist aufgebaut wie im bekannten Stand der Technik und weist einen Lageraußenring und einen Lagerinnenring und dazwischen Wälzkugeln auf, die die Lenksäule abstützen und drehbar lagern. Im Lagerzwischenraum ist eine rheologisch aktive Substanz eingelagert. Zur Erhöhung der Viskosität wird ein magnetisches Feld angelegt, wodurch sich der Kraftschluss zwischen den Lagerringen verändert.

Versuche der Anmelderin, ein solches Lager als Kupplung zu verwenden, haben zu keinem brauchbaren Ergebnis geführt. Wälzlager müssen ein geringes Spiel aufweisen, um die erforderliche Tragkraft und Laufruhe zu ermöglichen und um ein Ausschlagen und damit hohen Verschleiß zu verhindern. Bei einem herkömmlichen und für Lenkungen typischen Wälzlager mit einem Innendurchmesser von 30 mm und einem Außendurchmesser von 42 mm und Wälzkugeln von ca. 4 mm Durchmesser weist das Wälzlager insgesamt etwa ein fertigungsbedingt streuendes Spiel von 6 bis 20 µm auf (Radiale Lagerluft, Toleranzklasse "Normal" bzw. P5). Das radiale Laufspiel auf jeder radialen Seite der Wälzkugel beträgt dann die Hälfte davon, bewegt sich also zwischen 3 µm und 10 µm. Ein größeres Laufspiel beeinträchtigt die Tragkraft, erhöht das Laufgeräusch und führt zu einem erheblichen erhöhten Verschleiß.

Da magnetorheologische Fluide magnetisch polarisierbare Partikel mit maximalen Durchmesser von meist 10 µm aufweisen, hat sich nun gezeigt, dass schon bei Zugabe von einem Tropfen eines magnetorheologischen Fluides ein solches Wälzlager auch ohne Anlegen eines magnetischen Feldes und ohne Traglast sofort blockiert. Das liegt daran, dass in magnetorheologischen Fluiden auch ohne Anlage eines äußeren Magnetfeldes ein Partikel mit 10 µm Durchmesser nicht durch einen Spalt mit 3 µm durchgepresst/durchgewalzt werden kann. Zusätzlich bilden sich auch oder gerade deswegen Agglomerate oder Ketten von zwei oder mehr Partikeln, sodass eine Blockade des Wälzlagers auch ohne äußeres Feld auftreten kann. Im Normalzustand wirkt immer eine Traglast auf das Lager (Radial- oder Axialkraft), wodurch sich das Laufspiel der unter Last stehenden Wälzkörper bis gegen 0 verringert und hohe Flächenpressungen auftreten, wodurch das Wälzlager mechanisch blockieren muss, da dann selbst die kleinsten Partikel mit 1 µm Durchmesser nicht mehr zwischen dem Wälzkörper und der Läufbahn hindurch gehen können. Das Lager wird unbrauchbar und/oder defekt und die Partikel verklemmen mechanisch im Laufspalt. Hierbei ist es auch egal, wenn Wälzlager mit übergroßem Grundlaufspiel, z.B. SKF Baureihe C5 verwendet werden, abgesehen davon, dass ein vergrößertes Lagerspiel die Tragkraft verringert und die Lebensdauer massiv senkt.

Durch das stetige Abrollen der Wälzkörper auf der Laufläche im Normalbetrieb, d.h. mit Radial- oder Axiallast, ergeben sich teilweise sehr hohe Flächenpressungen auf der Laufläche, welche die dazwischenliegenden Metallpartikel (> 99% reines Eisen) des magnetorheologischen Fluides platt schleifen. Außerdem kann die Beschichtung der Partikel zum Schutz gegen Abrasion, Sedimentation, Agglomeration beschädigt werden. Weiterhin können auch die Laufflächen beschädigt werden. In der Praxis hat sich gezeigt, dass die so veränderten Partikel dann auch ohne Magnetfeld mechanisch zusammen kleben bzw. verklumpen, wodurch die magnetorheologische Flüssigkeit unbrauchbar wird. Dies geschieht schon bei geringen mechanischen Pressungen der Partikel. Zusätzlich lassen sich die so gebildeten Partikelklumpen selbst bei großen Laufspielen nicht mehr zwischen dem Wälzkörper und der Laufbahn hindurchdrücken und das Lager blockiert.

Außerdem werden konventionelle Wälzlager ja schließlich abgedichtet, um den Eintritt von Staub und harten Partikeln zu verhindern und damit den Verschleiß zu verringern.

Gleiches gilt für die DE 10 2006 034 966 A1, welches ein Wälz- oder Linearlager nach dem Stand der Technik mit verbesserter Lokalisierung des Schmierstoffes durch MR-Flüssigkeit zeigt.

Mit der US 2008/0053776 ist ein Drehmomentkupplung bekannt geworden, bei der magnetorheologische Flüssigkeit zwischen die abrollenden (kämmenden) Zahnräder gegeben und mit einem Magnetfeld beaufschlagt wird. Dadurch soll ein übertragbares Moment von bis zu 1.500 Nm moduliert werden. Damit solche Kräfte/Momente übertragen werden können, müssen sich die Zahnflanken berühren bzw. das Zahnradspiel geht auch hierbei gegen 0, wodurch die dazwischenliegenden MRF-Partikel durch die hohe Flächenpressung, wie zuvor beim Wälzlager der DE 10 2004 009 906 B3 beschrieben, beschädigt werden. Bzw. es können die Zahnflanken wegen der Partikelgröße und der Partikelanhäufung (Klumpenbildung) ohne Magnetfeld verklemmen und blockieren. Abhängig von der Last (vom Moment) ändern sich bei US 2008/0053776 die Flächenpressung und das Flankenspiel stetig.

Bei einer bekannten magnetorheologischen Kupplung mit zwei gering voneinander beabstandeten Kupplungsscheiben können sich die beiden im geeigneten Abstand angeordneten Kupplungsscheiben ohne Magnetfeld zunächst relativ frei gegeneinander drehen. Abhängig vom Schlupf der Kupplungsscheiben kann aber auch im feldfreien Zustand durch Scherung des MRF ein gewisses Grundmoment übertragen werden. Wenn ein Magnetfeld senkrecht zu den Kupplungsscheiben aktiviert wird, verkettet sich das magnetorheologische Fluid zwischen den Kupplungsscheiben und die beiden Kupplungsscheiben werden miteinander gekoppelt. Die Stärke des übertragbaren Drehmoments hängt von verschiedenen Parametern ab, so z.B. von dem Wirkabstand bzw. dem Momenteneinleitungsabstand, der Wirkfläche, der Anzahl der Kupplungsscheiben, der Relativdrehzahl bzw. dem Schlupf sowie von dem magnetorheologischen Fluid und insbesondere auch von der Stärke des Magnetfeldes. Wird das maximal übertragbare Drehmoment überschritten, so sinkt das übertragbare Drehmoment nicht auf Null ab, sondern verbleibt etwa bei seinem maximal möglichen Wert, da auseinander gerissene Ketten der Partikel des magnetorheologischen Fluides sich sofort neu bilden und somit wieder wirksam sind.

Eine weitere MRF kupplung ist aus DE 10 2007 028 990 bekannt.

MRF Kupplungen nach dem Stand der Technik benötigen zum Erreichen hoher übertragbare Momente von z.B. größer 50 Nm oder mehr große Kupplungsscheiben mit einem Durchmesser größer 150 mm. Daraus ergeben sich Schwierigkeiten durch die Auszentrifugierung der ferromagnetischen Partikel aufgrund des Dichteunterschiedes gegenüber dem Trägermedium. Das Fluid und die ferromagnetischen Partikel können sich entmischen.

Ein erheblicher Vorteil magnetorheologischer Kupplungen besteht darin, dass der Verschleiß reduziert wird. Die Belastung erfolgt nicht nur auf den äußeren Oberflächen der Kupplungsscheiben, sondern die Energie wird im gesamten Flüssigkeitsvolumen aufgenommen.

Nachteilig an den bekannten magnetorheologischen Kupplungen sind die hohe erforderliche magnetische Feldstärke und eine gewisse Baugröße, die sich aus den Parametern Wirkdurchmesser, Wirkfläche und Anzahl der Scheiben ergibt. Daraus resultiert ein entsprechendes Baugewicht, um die entsprechenden Drehmomente übertragen zu können, was ein schlechtes Drehmoment/Gewichts-Verhältnis bewirkt. Starke, von einer elektrischen Spule erzeugte Magnetfelder benötigen auf Dauer viel elektrische Energie, was ebenfalls nicht gewünscht ist.

Es ist deshalb die Aufgabe der vorliegenden Erfindung, eine magnetorheologische Übertragungsvorrichtung zur Verfügung zu stellen, welche im Vergleich zum Stand der Technik die Übertragung von höheren Kräften oder Momenten bei gleichzeitig möglicherweise kleinerer Bauform mit geringem Verschleiß ermöglicht.

Diese Aufgabe wird gelöst durch eine Vorrichtung mit den Merkmalen des Anspruchs 1 und durch ein Verfahren mit den Merkmalen des Anspruchs 22. Bevorzugte Weiterbildungen der erfindungsgemäßen magnetorheologischen Übertragungsvorrichtung sind Gegenstand der Unteransprüche. Weitere Vorteile und Merkmale der vorliegenden Erfindung ergeben sich aus der Beschreibung der Ausführungsbeispiele.

Eine erfindungsgemäße magnetorheologische Übertragungsvorrichtung weist wenigstens zwei koppelbare Komponenten auf, deren Kopplungsintensität beeinflussbar ist. Zur Beeinflussung der Kopplungsintensität ist wenigstens ein Kanal vorgesehen. Der Kanal enthält wenigstens teilweise wenigstens ein durch ein Magnetfeld beeinflussbares magnetorheologisches Medium mit magnetisch polarisierbaren Partikeln. Wenigstens eine Magnetfelderzeugungseinrichtung ist zur Erzeugung wenigstens eines Magnetfeldes in dem Kanal vorgesehen, um mit dem Magnetfeld das magnetorheologische Medium in dem Kanal zu beeinflussen. In bzw. an dem Kanal ist wenigstens ein Drehkörper vorgesehen. Ein freier Abstand zwischen dem Drehkörper und der Komponente ist wenigstens zehnmal so groß wie ein typischer mittlerer Durchmesser der magnetisch polarisierbaren Partikel in dem magnetorheologischen Medium. Zwischen dem Drehkörper und wenigstens einer Komponente ist wenigstens ein spitzwinkliger und das magnetorheologische Medium enthaltender bzw. sich bildender Bereich vorgesehen. Der Kanal oder wenigstens ein Teil davon ist mit dem Magnetfeld der Magnetfelderzeugungseinrichtung beaufschlagbar ist, um wenigstens einen Teil der Partikel wahlweise zu verketten und mit dem Drehkörper zu verkeilen oder freizugeben.

Insbesondere sind die beiden Komponenten wahlweise und gesteuert miteinander koppelbar.

Unter dem Begriff Kopplungsintensität werden im Sinne dieser Anmeldung die Kopplungskraft und/oder das Kopplungsmoment zwischen den beiden Komponenten verstanden. Wird z.B. eine lineare Kraftübertragung gewünscht, so entspricht die Kopplungsintensität der Kopplungskraft. Wenn ein Drehmoment übertragen werden soll, wird mit der Kopplungsintensität das Kopplungsmoment gemeint.

Vorzugsweise ist durch das Feld die Viskosität des magnetorheologischen Mediums veränderbar, wodurch die notwendige Verdrängungsarbeit zur Relativbewegung der relativ zueinander bewegbaren Komponenten und/oder Drehkörper beeinflussbar ist.

Unter Verdrängungsarbeit wird auch die Verdrängungskraft verstanden, die zur Verdrängung des Mediums bei einer Relativbewegung nötig ist.

Es ist bevorzugt, dass der wenigstens eine Drehkörper zwischen den beiden Komponenten angeordnet ist. Es ist aber auch möglich, dass eine der Komponenten als Drehkörper ausgebildet ist, der wenigstens teilweise an oder in dem Kanal vorgesehen ist.

Erfindungsgemäße magnetorheologische Übertragungsvorrichtungen haben viele Vorteile. Ein erheblicher und überraschender Vorteil der erfindungsgemäßen magnetorheologischen Übertragungsvorrichtung resultiert aus der erheblich verstärkten Wirkung des Magnetfeldes der Magnetfelderzeugungseinrichtung in dem Kanal. Der spitzwinklige und das Medium enthaltende Bereich wirkt als Hebel und somit quasi wie eine starke mechanische Hebelübersetzung, wobei der Hebel die Wirkung des Magnetfeldes um ein Mehrfaches erheblich verstärkt. Dadurch kann entweder die Feldstärke der Magnetfelderzeugungseinrichtung bei gleichbleibender Wirkung reduziert werden oder aber der Effekt des Magnetfeldes wird bei gleichbleibender Feldstärke verstärkt oder es wird sogar die Wirkung bei reduzierter Feldstärke deutlich erhöht. Durch den spitzwinkligen und das Medium enthaltenden Bereich wird die Wirkung insbesondere um ein Mehrfaches erhöht, wenn das Magnetfeld auf das Medium einwirkt. Insbesondere wirkt das Magnetfeld wenigstens zeitweise auf den spitzwinkligen und das magnetorheologische Medium enthaltenden bzw. sich bildenden Bereich ein.

Dadurch, dass der Drehkörper mit einem erheblichen freien Abstand gegenüber der wenigstens einen Komponente angeordnet ist, kann ein makroskopischer Keil entstehen, der zur Übertragung starker Kupplungs- oder Bremsmomente dienen kann. Durch die völlig überraschende Vervielfachung der Wirkung kann erheblich Bauvolumen eingespart werden. Der ausgenutzte Effekt beruht auf der Keilbildung (Haufenbildung) und nicht nur dem magnetorheologischen Verketten einzelner Partikel. Die typische Reaktionszeit zur Keilbildung benötigt etliche Millisekunden, während das Verketten einzelner Partikel gemäß dem MRF-Effekt schon innerhalb ca. 1 Millisekunde erfolgt. Diese mehrfach längere Zeitdauer ist der Keilbildung geschuldet. Eine solche erhebliche Verstärkung der Wirkung war nicht erwartet worden. Die längere Reaktionszeit von z.B. 5, 10 oder 20 Millisekunden ist in vielen Anwendungsfällen mehr als ausreichend.

Der Kanal kann auch ein Zwischenraum oder ein nach 4 Seiten offener Raum sein.

Als spitzwinkliger Bereich des Kanals wird jener Kanalbereich definiert, der durch die Form von Drehkörper und Komponenten in wenigstens einem Querschnitt annähernd spitzwinklig aussieht. Die Seiten des Bereichs müssen nicht gerade sein, sie können auch gekrümmt sein und/oder eine andere Kontur aufweisen. Dabei definiert der spitzwinklige Bereich jenen Teil des Kanals, in dem Drehkörper und Komponenten insbesondere den kleinsten Abstand zueinander haben bzw. sich berühren sowie den angrenzenden Bereich, in dem sich die Oberflächen von Drehkörper und Komponenten voneinander entfernen.

Unter der Wirkung eines Magnetfeldes bildet sich der spitzwinklige und das magnetorheologische Medium enthaltende Bereich aus, in dem eine erheblich erhöhte Viskosität vorliegt.

Die Erfindung ermöglicht ein gutes Drehmoment zu GewichtsVerhältnis, welches größer als 100 Nm/kg sein kann.

Vorzugsweise wird ein Drehkörper durch eine Relativgeschwindigkeit zu wenigstens einer Komponente in eine Drehbewegung versetzt. Dabei ist es möglich, dass die Umfangsgeschwindigkeit des Drehkörpers gleich der Relativgeschwindigkeit zu der Komponente ist. Es ist aber auch möglich, dass die Umfangsgeschwindigkeit des Drehkörpers auf seiner äußeren Oberfläche größer oder kleiner als die Relativgeschwindigkeit ist. Insbesondere ist es möglich, dass die Umfangsgeschwindigkeit des Drehkörpers auf seiner äußeren Oberfläche geringer ist als die Relativgeschwindigkeit des Drehkörpers zu der Komponente.

Der Drehkörper kann im Wesentlichen rotationssymmetrisch um wenigstens eine Drehachse ausgebildet sein. Ebenso ist es möglich, dass der Drehkörper zu mehreren Drehachsen rotationssymmetrisch ausgebildet ist. Beispielsweise kann der Drehkörper als Kugel oder Ellipsoid ausgebildet sein. Möglich ist es auch, dass der Drehkörper als Zylinder, Rolle oder allgemein als Rollkörper ausgestaltet ist. Insbesondere eine etwa zylindrische Ausgestaltung hat sich als vorteilhaft erwiesen, da sich beispielsweise bei einem zylindrischen Drehkörper über die gesamte Breite des Drehkörpers der spitzwinklige und das Medium enthaltende Bereich ausbildet, der so im Wesentlichen keilförmig ausgestaltet ist. Bei diesen und anderen Ausgestaltungen weist der spitzwinklige Bereich eine Keilform auf.

Es ist aber nicht nötig, dass der Drehkörper rotationssymmetrisch ausgebildet ist. Auch Drehkörper mit elliptischen oder eiförmigen Querschnitten oder Drehkörper mit Einbuchtungen wie Golfbälle oder mit regelmäßigen oder unregelmäßigen Ein und/oder Ausbuchtungen können vorteilhaft verwendet werden. Die Oberfläche der Drehkörper kann glatt ausgestaltet sein, muss es aber nicht. Da die Drehkörper nicht zur Lagerung und Abstützung der Komponenten gegeneinander verwendet werden, ist eine symmetrische und/oder glatte Oberfläche nicht notwendig. Vorteilhaft können sogar Drehkörper mit rauer und/oder unregelmäßiger Oberfläche sein, da der Keileffekt verstärkt wird. Ein erhöhter Verschleiß tritt nicht auf, da die Drehkörper nicht zur Lagerung und Übertragung von Tragkräften eingesetzt werden.

Die Verstärkung der Wirkung erfolgt nicht nur durch eine Verstärkung oder Bündelung des Magnetfeldes, sondern vor allem auch durch die vor den Drehkörpern oder Rollen angehäuften Partikel und deren Verdichtung. Wegen des Magnetfeldes können die Partikel nicht weg und verdichten so schneller zu einem Keil. Der Keil ist von außen einfach per Schalter steuerbar. Der Vorteil bei magnetorheologischen Fluiden wie MRF ist, dass durch das Aufheben des Magnetfeldes sich der Keil wieder lösen kann. Mit dem Magnetfeld kann - ohne mechanische Bewegung oder Krafteinleitung - der Keil beeinflusst werden. Zur gezielten Beeinflussung und sicheren Steuerung hat es sich als vorteilhaft herausgestellt, dass der freie Abstand zwischen dem Drehkörper und der Komponente größer als ein mehrfaches des Partikeldurchmessers ist.

Der Durchmesser der Partikel des magnetorheologischen Mediums beträgt insbesondere zwischen 1 µm und 10 µm. Der typische mittlere Durchmesser der Partikel des magnetorheologischen Mediums ist der arithmetisch gemittelte Durchmesser der Partikel die größer sind als das kleinste Prozent und die kleiner sind als das größte Prozent. In der Regel entspricht dieser Wert dem Mittelwert der Durchmesser des größten und des kleinsten Partikels, im gewählten Beispiel also 5,5 µm. Wenn aber beispielsweise eine sehr geringe Zahl noch kleinerer Partikel vorhanden ist, ändert das nichts an dem so bestimmten typischen mittleren Durchmesser. Das gleiche gilt, wenn z.B. einzelne Partikel mit 10,5 µm oder 11 µm Durchmesser enthalten sein sollten.

Vorzugsweise beträgt der freie Abstand zwischen dem Drehkörper und der Komponente mehr als 30 µm und insbesondere weniger als 300 µm. Der typische mittlere Durchmesser der Partikel liegt vorzugsweise zwischen 3 µm und 7 µm. Vorzugsweise beträgt der freie Abstand zwischen dem Drehkörper und der Komponente mehr als 70 µm und insbesondere weniger als 250 µm.

Die Anmelderin behält sich vor, Schutz für solche magnetorheologische Übertragungsvorrichtungen zu beanspruchen, bei denen ein freier Abstand zwischen dem Drehkörper und der Komponente größer ist als der Durchmesser des typischen größten magnetisch polarisierbaren Partikels. Insbesondere ist der freie Abstand größer als der doppelte Durchmesser des typischen größten magnetisch polarisierbaren Partikels und kann somit kleiner sein als bei der ansonsten gleichen zuvor beschriebenen erfindungsgemäßen magnetorheologischen Übertragungsvorrichtungen.

Vorteilhafterweise verkeilt der spitzwinklige Bereich bei Anlage eines Magnetfeldes die zwei sich ohne Magnetfeld frei zueinander bewegbaren Komponenten. Ein mechanischer Keil in Form eines separaten festen Teils ist dafür nicht erforderlich.

Vorzugsweise ist der spitzwinklige Bereich zwischen dem Köper und einer Komponente so vorgesehen, dass sich der spitzwinklige Bereich in Richtung der Relativbewegung der Komponente relativ zu dem Drehkörper verjüngt. Wälzt ein zylindrischer Drehkörper auf einer flachen Oberfläche einer Komponente ab, so bildet sich der spitzwinklige Bereich in Keilform vor dem Drehkörper. Durch die Verkettung der Partikel in dem Medium entsteht ein sich insgesamt verkettender Keil, der die Relativbewegung des Drehkörpers zu der Komponente hemmt.

Besonders bevorzugt ist der und insbesondere jeder Drehkörper als separates Teil zwischen der ersten und der zweiten Komponente ausgebildet. Dann ist es bevorzugt, dass die eine Komponente als äußere Komponente die andere Komponente als innere Komponente umgibt. Beispielsweise kann eine (Antriebs-) Welle als innere Komponente vorgesehen sein. Die andere bzw. äußere Komponente kann beispielsweise zum Bremsen dienen und die Welle radial umgeben. Zwischen der Welle und der äußeren Komponente können die Drehkörper vorgesehen sein. Es hat sich herausgestellt, dass zur Erzielung des Keileffektes sich um ihre eigene Achse drehende Drehkörper erheblich besser sind. Ausgearbeitete Lagerschalen sind nicht nötig. Die Übertragung eines Kupplungs- oder Bremsmomentes funktioniert unabhängig von der Qualität der Abrollflächen.

Zur Lagerung der beiden Komponenten ist vorzugsweise wenigstens ein separates Wälzlager vorgesehen. Insbesondere werden die beiden Komponenten über wenigstens zwei zusätzliche Wälzlager drehbar gelagert und vorzugsweise drehbar gegenüber einander gelagert. Die Drehkörper sorgen mit dem Keileffekt für die Übertragung der gewünschten Drehmomente, während das oder die Wälzlager zur definierten Führung und Abstützung der beiden Komponenten und dem gleichbleibenden Laufspalt sorgen. Aufgrund des erheblichen freien Abstandes bzw. aufgrund des Spiels der Drehkörper gegenüber den Komponenten kann ohne den Einsatz von Wälzlagern ein Verkippen der Komponenten zueinander auftreten.

In allen Ausgestaltungen ist vorzugsweise der freie Abstand mindestens zehnmal so groß ist wie der größte typische Partikeldurchmesser. In bestimmten Ausgestaltungen hat sich ein freier Abstand zwischen etwa dem fünffachen und insbesondere dem zehnfachen und dem zwanzigfachen größten typischen Partikeldurchmesser als vorteilhaft herausgestellt. Bei größeren freien Abständen wird das maximal übertragbare Drehmoment wieder reduziert, da der Keileffekt nachlässt. Bei zu geringen freien Abständen kann es auch ohne Magnetfeld zu einer Blockade kommen. Außerdem kann dann nicht immer ein Lösen des Keils nach dem Abschalten des Magnetfeldes gewährleistet werden.

Unter dem mittleren Partikeldurchmesser wird das arithmetische Mittel aus minimalem und maximalem Partikeldurchmesser verstanden. Die meisten MRF weisen magnetisch polarisierbare Partikel auf, die eine Größenverteilung zwischen etwa 1 µm und 10 µm aufweisen. Der mittlere Partikeldurchmesser ist bei diesem Beispiel 5,5 µm. Bei variablen Größenverteilungen wird unter dem größten typischen Partikeldurchmesser ein Partikeldurchmesser verstanden, den nur weniger als 1% der Partikel überschreiten. Der größte typische Partikeldurchmesser ist im genannten Beispiel etwas geringer als 10 µm, sodass hier von 10 µm als größtem typischen Partikeldurchmesser ausgegangen werden kann.

Vorzugsweise ist der freie Abstand größer als 1/500 und vorzugsweise größer als 1/250 und insbesondere größer als ein 1/100 und besonders bevorzugt größer als 1/50 eines Durchmessers wenigstens eines Drehkörpers und insbesondere ist der freie Abstand kleiner als 1/10 und insbesondere kleiner als 1/20 des Durchmessers des Drehkörpers.

Der freie Abstand ist vorzugsweise größer als 1/300 des Außendurchmessers der inneren Komponente und/oder größer als 1/500 des Innendurchmessers der äußeren Komponente. Vorzugsweise ist der freie Abstand größer als 30 µm und insbesondere kleiner als 200µm.

Bei allen Zahlenangaben sind vorzugsweise Variationen um +/- 20% möglich. Unter einem Partikel wird im Folgenden ein magnetisch polarisierbarer Partikel verstanden.

Werden übergroße Drehkörper und/oder Wellendurchmesser verwendet, können andere Abstände vorteilhaft sein. Ein Vorteil dieser magnetorheologischen Übertragungsvorrichtung mit wenigstens zwei koppelbaren Komponenten ist, dass die Keilbildung fertigungstolerant ist, d.h. z.B. fertigungs- und montagebedingte Unterschiede in Spalthöhen, Oberflächen, Abmessungen wie auch Wärmeausdehnungen oder lastbedingte Verschiebungen von Komponenten haben einen untergeordneten Einfluss auf diesen und verursachen vernachlässigbare Momenten- oder Kraftunterschiede.

Eine z.B. konstruktiv bedingte Änderung vom Spalt innerhalb gewisser Systemgrenzen kann auch z.B. durch Sensoren erkannt und durch Feldanpassung ausgeregelt werden.

In bevorzugten Ausgestaltungen ist der Drehkörper Teil der ersten oder der zweiten Komponente. Das bedeutet, dass der beispielsweise als Drehkörper ausgebildete Drehkörper Teil der ersten Komponente ist und auf der zweiten Komponente beispielsweise abrollt. Der Drehkörper kann aber auch ohne mechanische Verbindung zu beiden Komponenten sein.

In dem spitzwinkligen und zum Beispiel keilförmigen Bereich verketten sich bei Anlage eines äußeren Magnetfeldes die ferromagnetischen Partikel in dem Medium und führen zu einem örtlich festeren Gebilde, welches sich der weiteren Relativbewegung zwischen dem Drehkörper und der benachbarten Komponente widersetzt. Durch die Wälzbewegung des Drehkörpers können die Partikel im keilförmigen Teil in Bewegungsrichtung vor dem Drehkörper zusätzlich verdichtet werden. Diese Verdichtung kann aber je nach Gestaltung des Drehkörpers auch durch Nick-, Kipp- oder sonstige Bewegungen relativ zu einer Komponente erfolgen.

Rollt der Drehkörper beispielsweise auf der Oberfläche einer Komponente ab und bildet sich vor dem Drehkörper ein solcher spitzwinkliger Bereich aus, so werden durch die Drehbewegung des Drehkörpers von der äußeren Oberfläche Partikel in dem Medium mitgerissen und in Drehbewegung versetzt, wobei sich aber der sich verhärtende spitzwinklige Bereich einer solchen Drehbewegung stark widersetzt. Der spitzwinklige Bereich in Keilform führt zu einer Kraft auf den Drehkörper weg von der Komponente. Gegebenenfalls kann eine solche Kraft und eine daraus resultierende Bewegung auch für Feinjustagezwecke verwendet werden. Vorzugsweise kann durch den spitzwinkligen Bereich in Keilform eine rotative Bewegung in eine axiale Verschiebung des Drehkörpers umgewandelt werden, wenn das Magnetfeld aktiviert wird. Dadurch wird quasi ein Aufschwimmen des Drehkörpers durch die Partikel bewirkt. Es ist auch möglich den Drehkörper oder eine Komponente beispielsweise mit gewindeförmigen Einkerbungen zu versehen oder relativ zueinander schräg zu lagern, um die Wirkrichtung der resultierenden Kraft zu verändern oder die erreichbare Kraftübersetzung weiter zu erhöhen. Dadurch kann mit einer Art Gewindestange eine lineare Bewegung in eine Rotationsbewegung überführt werden. Durch Anlage eines Feldes wird die Relativbewegung gehemmt.

Es ist ebenso bevorzugt, dass der Drehkörper als separates Teil zwischen der ersten und der zweiten Komponente ausgebildet ist. Eine solche Ausgestaltung kann besonders vorteilhaft sein, da es zu zwei spitzwinkligen Bereichen bzw. keilförmigen Bereichen zwischen dem Drehkörper und den beiden Komponenten kommen kann. Liegt der Drehkörper auf der einen Seite praktisch an der ersten Komponente und an der anderen Seite praktisch an der zweiten Komponente an, so bilden sich auf beiden Seiten spitzwinklige Bereich, die dem Magnetfeld der Magnetfelderzeugungseinrichtung ausgesetzt werden. Dadurch wird die Wirkung erhöht. Dazu ist es nicht nötig, dass der Drehkörper vollständig an der ersten Komponente oder der zweiten Komponente anliegt. Dabei verbleibt zwischen dem Drehkörper und der jeweiligen Komponente ein geringer Spalt. Die Größe des Spaltes hängt unter anderem von den Eigenschaften des Mediums ab. Insbesondere kann die Größe des Spaltes wenigstens dem fünffachen und vorzugsweise wenigstens dem zehnfachen oder zwanzigfachen eines typischen oder mittleren Partikeldurchmessers entsprechen.

Die ferromagnetischen Partikel bestehen insbesondere aus Carbonyleisenpulver. Das Fluid kann z.B. ein Öl sein.

Möglich ist es auch, dass magnetorheologische und elektrorheologische Medien gemeinsam eingesetzt werden. Denkbar ist auch der Einsatz anderer Medien, die durch entsprechende Felder beeinflusst und beispielsweise verkettet werden. Ebenso möglich ist der Einsatz von Medien, welche ihre rheologischen Eigenschaften in Abhängigkeit von sonstigen physikalischen Größen wie beispielsweise Temperatur oder Schergeschwindigkeit verändern.

Der Kanal kann vollständig oder auch nur teilweise mit dem Medium gefüllt sein. Vorzugsweise ist zumindest der spitzwinklige Bereich des Kanals mit dem Medium gefüllt.

In allen Ausgestaltungen kann die erste und/oder zweite Komponente rotationssymmetrisch ausgebildet sein. Beispielsweise können die Komponenten jeweils als Scheiben oder zylindrische Körper ausgebildet sein, zwischen denen Drehkörper vorgesehen sind, um durch den Keileffekt die Wirkung des Magnetfeldes der Magnetfelderzeugungseinrichtung entsprechend zu erhöhen.

In allen Ausgestaltungen ist es bevorzugt, dass das Magnetfeld durch den Drehkörper und insbesondere im Wesentlichen quer zu der Relativbewegung der Komponenten zueinander und von einer Komponente zu der anderen Komponente wenigstens teilweise durch den Drehkörper verläuft. Eine solche Ausgestaltung hat sich als besonders wirkungsvoll herausgestellt, da die Wirkung des Magnetfeldes an den Übergangspunkten von dem Drehkörper auf die Wände des Kanals besonders stark ist. Abhängig vom wirkenden Magnetfeld ist es deshalb vorteilhaft, wenn der Drehkörper zumindest teilweise magnetisch leitfähig ist. Insbesondere ist wenigstens eine und insbesondere sind beide Komponenten und/oder der wenigstens eine Drehkörper zumindest teilweise aus einem ferromagnetischen Material. Die Permeabilitätszahl ist vorzugsweise größer als 500. Die auch relative Permeabilität genannte Permeabilitätszahl des Materials kann auch 1000, 2000 oder mehr betragen. Möglich sind beispielsweise Drehkörper aus einem ferromagnetischen Stahl, wie ST37.

Durch ein gedämpftes magnetisches Wechselfeld kann eine Entmagnetisierung des Materials erfolgen, damit ohne Restfeld ein niederes Grundmoment erreicht wird.

In allen Ausgestaltungen ist es bevorzugt, dass die Magnetfelderzeugungseinrichtung wenigstens einen Dauermagneten und/oder wenigstens eine Spule umfasst. Möglich ist auch der Einsatz eines oder mehrerer Dauermagneten und einer oder mehrerer elektrischer Spulen.

Möglich und bevorzugt ist es, die Magnetisierung des Dauermagneten durch wenigstens einen magnetischen Impuls einer elektrischen Spule dauerhaft zu verändern. Bei einer solchen Ausgestaltung wird der Dauermagnet durch magnetische Impulse der Spule so beeinflusst, dass die Feldstärke des Dauermagneten dauerhaft verändert wird. Dabei kann die dauerhafte Magnetisierung des Dauermagneten durch den magnetischen Impuls der Magnetfelderzeugungseinrichtung auf einen beliebigen Wert zwischen Null und der Remanenz des Dauermagneten eingestellt werden. Auch die Polarität der Magnetisierung ist veränderbar. Ein magnetischer Impuls zur Einstellung einer Magnetisierung des Dauermagneten ist insbesondere kürzer als 1 Minute und vorzugsweise kürzer als 1 Sekunde und besonders bevorzugt beträgt die Länge des Impulses weniger als 10 Millisekunden.

Als Effekt eines Impulses bleibt die Form und Stärke des Magnetfeldes in dem Dauermagneten dauerhaft erhalten. Die Stärke und Form des Magnetfeldes kann durch zumindest einen magnetischen Puls der Magnetfelderzeugungseinrichtung geändert werden. Durch ein gedämpftes magnetisches Wechselfeld kann eine Entmagnetisierung des Dauermagneten erfolgen.

Als Material für solch einen Dauermagneten mit veränderbarer Magnetisierung eignet sich beispielsweise A1NiCo, es können aber auch andere Materialien mit vergleichbaren magnetischen Eigenschaften eingesetzt werden. Es ist zudem möglich, anstelle eines Dauermagneten den gesamten magnetischen Kreis bzw. Teile von ihm aus einer Stahllegierung mit starkem Restmagnetismus (hoher Remanenz) herzustellen.

Möglich ist es, mit dem Dauermagneten ein dauerhaftes statisches Magnetfeld zu erzeugen, welches durch ein dynamisches Magnetfeld der Spule überlagert werden kann, um die gewünschte Feldstärke einzustellen. Dabei kann durch das Magnetfeld der Spule der aktuelle Wert der Feldstärke beliebig verändert werden. Möglich ist auch der Einsatz zweier getrennt ansteuerbarer Spulen.

In allen Ausgestaltungen ist es bevorzugt, wenigstens eine Steuereinrichtung vorzusehen. Möglich ist auch der Einsatz eines Energiespeichers wie beispielsweise eines Kondensators zur Speicherung zumindest eines Anteils der benötigten Energie. Wenigstens ein Sensor oder mehrere Sensoren können zur Detektion relevanter Daten wie beispielsweise der Relativgeschwindigkeit der Komponenten zueinander oder der vorherrschenden Feldstärke und dergleichen dienen. Möglich ist es auch, als Sensor einen Temperatursensor einzusetzen, der z. B. bei Überschreiten vorbestimmter Temperaturbedingungen einen Alarm auslöst. Es kann vorteilhaft ein Drehwinkelgeber eingesetzt werden, um jederzeit Daten über die Winkelposition der Komponenten zueinander zu haben.

In allen Ausgestaltungen ist es bevorzugt, dass der Dauermagnet zumindest teilweise aus einem hartmagnetischen Material besteht, dessen Koerzitivfeldstärke größer als 1kA/m und insbesondere größer als 5kA/m und vorzugsweise größer als 10kA/m ist.

Der Dauermagnet kann zumindest teilweise aus einem Material bestehen, welches eine Koerzitivfeldstärke kleiner als 1000 kA/m und vorzugsweise kleiner als 500 kA/m und besonderes bevorzugt kleiner als 100 kA/m aufweist.

Vorzugsweise kann eine erfindungsgemäße magnetorheologische Übertragungsvorrichtung als Teil eines Lagers, einer Bremse, einer Kupplung eines Bedien- oder Steuerknopfes oder eines Stoßdämpfers oder dergleichen ausgebildet sein. Möglich ist auch der Einsatz als Lenkradschloss, wobei durch einen Dauermagneten eine dauerhafte Generierung der erforderlichen Feldstärke gewährleistet ist.

Der Drehkörper und wenigstens eine Komponente können sich an zumindest einem Punkt oder zumindest einer Linie berühren. Es ist möglich und bevorzugt, dass der Drehkörper relativ zu zumindest einer Komponente in Ruhe ist.

Vorzugsweise kann sich der Drehkörper relativ zu zumindest einer Komponente, beispielsweise in Form einer Dreh- oder Kippbewegung, bewegen.

Die Feldstärke kann abhängig von dem jeweiligen Abstand zwischen Drehkörper und Komponenten einen starken Gradienten aufweisen.

Die Feldstärke erhöht sich vorzugsweise im spitzwinkligen Bereich zwischen Drehkörper und Komponenten zum Bereich mit dem geringsten Abstand hin.

Mit der Erfindung ist beispielsweise auch eine Diebstahlsicherung in Form einer Lenkradsperre zum Schutz vor Fahrzeugdiebstahl möglich. Dabei wird die Lenksäule beispielsweise durch ein starkes Erhöhen des Drehmomentes blockiert. Dazu kann ein Dauermagnet ein dauerhaftes Magnetfeld erzeugen, wodurch eine Relativbewegung der Lenkstange zur Lenksäule erheblich erschwert wird. Bei konventionellen Lenkradschlössern wird bei einer Überlast der Sperrbolzen abgeschert, wonach ein freies Bewegen der Lenkstange möglich ist. Im Unterschied dazu bleibt bei einer erfindungsgemäßen Lösung die vorgesehene Kraft erhalten, auch wenn sie einmal überschritten wurde.

Eine erfindungsgemäße magnetorheologische Übertragungsvorrichtung in Form z.B. einer Kupplung oder Bremse oder dergleichen hat bei erheblich geringerem Raumbedarf eine erheblich größere Wirkung. Das Verhältnis des Bauraumbedarfes zum Stand der Technik kann den Faktor 10 erreichen oder übersteigen. Die Verwendung eines magnetorheologischen Fluides als Medium bei einer erfindungsgemäßen magnetorheologischen Übertragungsvorrichtung erlaubt die kostengünstige Herstellung einer Kupplung oder einer Bremse oder dergleichen. Der Wartungsbedarf kann erheblich reduziert werden, da wenige und einfache Bauteile eingesetzt werden. Gegebenenfalls kann die Wartung durch einfachen Austausch der magnetorheologischen Flüssigkeit durchgeführt werden. Der Aufbau ist einfach und robust und es werden keine Stromdurchführungen benötigt. Außerdem ist der Energiebedarf geringer als beim Stand der Technik, denn der Keileffekt trägt erheblich zur Beeinflussung der Relativbewegung der Komponenten bei. MRF-Bremsen bzw. MRF- Kupplungen mit einem Drehmoment/Gewichts-Verhältnis von >100 Nm/kg werden dadurch möglich.

Bei magnetorheologischen Kupplungen bzw. Bremsen nach dem Stand der Technik bewegen sich die Magnetfeldpole relativ zueinander und erzeugen in der dazwischenliegenden MR Flüssigkeit Scherkräfte (Direct Shear Mode). Die Scherkräfte variieren je nach Magnetfeld. Kein Magnetfeld bedeutet keine bzw. geringe Scherkräfte (keine Kettenbildung im MRF), maximales Magnetfeld bedeutet maximale Scherkräfte und damit maximale Bremskraft bzw. Bremsmoment. Vereinfacht gesagt sind Magnetfeld und Scherkräfte proportional.

Bei der vorliegenden Erfindung kann durch entsprechende Gestaltung der Einzelkomponenten, Dimensionierung und Feldeinbringung ein davon abweichendes und sehr vorteilhaftes Verhalten geschaffen werden. Dieses vorteilhafte Verhalten äußert sich dahingehend, dass zum Halten der spitzwinkligen Ausbildung bzw. des MR-Fluid Keils ein wesentlich geringeres Magnetfeld und damit eine geringere Stromstärke als zum erstmaligen Erzeugen des Keils benötigt wird. Dies kommt daher, dass die Partikelanhäufung, ist sie erstmals angehäuft und durch die dieser Erfindung zugrundeliegenden speziellen Bewegungen unter Einfluss eines richtig eingebrachten Magnetfeldes quasi mechanisch verdichtet, nicht mehr so leicht zerfällt. Dies hat zur Folge, dass z.B. nach einer entsprechenden Zeit zum Erreichen dieses Zustandes ein Bremsmoment mit dem Bruchteil des Magnetfeldes bzw. an elektrischer Leistung (Spulenstrom) gehalten werden kann, was energietechnisch vorteilhaft ist.

Werden Kupplungen mit magnetorheologischen Flüssigkeiten nach dem Stand der Technik über das maximal übertragbare Kupplungsmoment hinaus beansprucht, beginnen einzelne Partikelketten abzureißen, wodurch sich ein Schlupf bzw. ein Durchrutschen einstellt. Das maximale Kupplungsmoment bleibt aber erhalten, es nimmt mitunter sogar leicht zu, die Kupplung löst sich nicht. Je nach Anwendung kann dies unerwünscht sein, so z.B. wenn sich ein Bohrmaschinenbohrer beim Bohren verklemmt.

Bei der vorliegenden Erfindung kann durch entsprechende Gestaltung der Einzelkomponenten, Dimensionierung und Feldeinbringung ein davon abweichendes und sehr vorteilhaftes Verhalten geschaffen werden. Dieses vorteilhafte Verhalten äußert sich dahingehend, dass beim Überschreiten einer maximalen Kraft zwischen den sich bewegenden Teilen der vom Magnetfeld erzeugte Keil (Materialanhäufung) schlagartig durch den Spalt gepresst wird (Material verdrängt) und die Kraft damit eingehend schlagartig abnimmt. Aufgrund der daraus resultierenden Relativbewegung und der hohen anliegenden Kraft bildet sich kein neuer Keil, wodurch die Relativkraft niedrig bleibt. Bei Überlastkupplungen ist dieses Verhalten sehr vorteilhaft. Über das Magnetfeld kann die maximale Kraft (Auslösekraft) bzw. das maximale Moment (Auslösemoment) voreingestellt werden.

Weiterhin werden Entmischungs-, Sedimentations- und Fliehkraftprobleme zuverlässig vermieden, da durch die rotierenden Drehkörper eine ständige Vermischung der Partikel in dem Medium erzielt wird.

Aufgrund der erheblich höheren übertragbaren Momente und Kräfte können Kupplungen, Bremsen oder dergleichen mit wesentlich kleineren Durchmessern realisiert werden. Aufgrund der geringen MRF Kanalhöhe und der Drehbewegung der Drehkörper ist ein Entmischen bei der vorliegenden Erfindung praktisch nicht relevant.

Die Erfindung kann vielfältig eingesetzt werden, so z.B. in Prothesen als Gelenk für rotierende Komponenten und als Dämpfer bei einer Linearbewegung. Der Einsatz ist auch an einer Fahrzeugtür möglich, um variable Anschläge oder ein definiertes Aufstehen der Tür zu erlauben. Der Einsatz ist ebenso als Blinkerhebel an Fahrzeugen oder als Überlastfunktion an Werkzeugmaschinen möglich, um beim Überschreiten eines Grenzdrehmomentes ein genaues Lösen der Kupplung zu ermöglichen.

Eine erfindungsgemäße Kupplung kann eingesetzt werden, um Drehmoment oder Drehzahl am Abtrieb unabhängig vom Antrieb zu halten, beispielsweise um die Drehzahl konstant zu halten oder ein bestimmtes Drehmoment nicht zu überschreiten. Gleichfalls kann die Verwendung auch bei Einsatzzwecken erfolgen, bei denen ein hohes Drehmoment übertragen werden soll, so z.B. bei der Drehmomentverteilung an einem Antriebsstrang.

Weitere Einsatzmöglichkeiten sind Kupplungen bei elektrischen Antrieben um eine Last gesteuert anzukuppeln, NC-Fräsmaschinen, Holzbearbeitungsmaschinen, Automatisierungsanlagen sowie die Verwendung bei Industrierobotern, Blechbearbeitungsmaschinen, Druckmaschinen, Textilmaschinen, Webmaschinen, Wickelvorrichtungen, Strohpressen, Ladewagen, elektrischen Fensterhebern, Garagentoren, Rollos etc. sowie in Schnellfräsen, Küchenmaschinen, Mühlen und dergleichen mehr.

Soll z.B. ein Medium wie z.B. Papier, Faden oder dergleichen mit gleichbleibender Zugspannung auf eine Rolle aufgewickelt werden, so kann dies mit der Erfindung durch Variation des Antriebs- oder Bremsmomentes entsprechend der Durchmesseränderung der Aufwickelrolle erzielt werden. Weitere Einsatzgebiete sind adaptive Bremsen bei Fitnessgeräten (z.B. Rotation: Fahrradtrainer; Laufband; Hebel bei Gewichtheben, Rudermaschine; Linearbewegung: Gewichte heben, die Klemmung der linearen Höhenverstellung von einem Sattel oder Bürostuhl oder der Längsverstellung einer Lenksäule oder eines Sitzes in einem Fahrzeug.

Auch bei einer dreidimensionalen Bewegung kann die Erfindung eingesetzt werden. So kann die Rotation und Pendelbewegung durch den MRF Keil beschränkt bzw. blockiert werden. Das wirkende Moment ist stufenlos einstellbar und es können Schaltzeiten im Bereich einiger Millisekunden erzielt werden. Der Aufbau ist einfach und es sind keine mechanisch bewegten Teile zum Variieren des Drehmoments nötig. Ein weiterer Vorteil ist, dass ein fast geräuschloser Betrieb möglich ist. Die Mehrkosten sind gering und eine erfindungsgemäße magnetorheologische Übertragungsvorrichtung kann betriebssicher ausgelegt werden, wenn beispielsweise ein Dauermagnet mit Remanenz für die Einstellung eines Magnetfeldes verwendet wird. Der Keileffekt verstärkt die Wirkung enorm, sodass ein kleinerer Bauraum erzielbar ist.

In allen Ausgestaltungen müssen die Drehkörper nicht glatt sein, sondern können raue oder unebene Oberflächen aufweisen.

Möglich ist auch die Verwendung der Erfindung als haptischer Drehknopf bzw. Haptikdrehknopf. Dadurch kann praktisch ein Drehknopf oder eine Art Potentiometer realisiert werden. Das Einsatzgebiet ist vielfältig und umfasst z.B. Regler für eine Kranbedienung oder dergleichen. Dabei kann die Drehung je nach Last schwergängiger gesteuert werden. Es kann auch in Abhängigkeit von der Lasthöhe gesteuert werden.

Interessant ist der Einsatz auch bei "Forcefeedback"- oder bei "Steer by wire"-Anwendungen. Auch der Einsatz an Bedienelementen in Fahrzeugen, Autoradios, Stereoanlagen etc. ist möglich.

In allen Ausgestaltungen ist es möglich, zusätzlich zu einer Dichtung mit einer Dichtlippe auch magnetische Dichtungen zur Abdichtung einer erfindungsgemäßen Vorrichtung einzusetzen. Dabei kann die Dichtung über einen Permanentmagneten erfolgen. Vorteile einer solchen Ausgestaltung sind kleinere Grundkräfte, die Verschleißfreiheit und die Zulässigkeit größerer Fertigungstoleranzen.

Außerdem liegt ein definiertes Überlastverhalten vor, da ein definiertes Durchbrechen erfolgt, wenn die Überlast überschritten wird. Es ist möglich eine solche Dichtung vor oder hinter einer erfindungsgemäßen Vorrichtung oder davor und dahinter einzusetzen.

Ein erheblicher Vorteil der Magnetdichtung ist die sehr geringe Reibung; es kann aber nötig sein, noch eine weitere Dichtung einzusetzen, da eine solche Dichtung gegebenenfalls nur MRF Partikel zurückhält und z.B. Öl als Basisflüssigkeit mit der Zeit durch den Spalt durchtreten lässt. Deshalb kann eine solche magnetische Dichtung als Vordichtung eingesetzt werden, um MRF-Partikel zurückzuhalten. Eine weitere z.B. klassische Dichtung dichtet dann nur noch das Trägermedium ab.

Eine Bewegung des Magneten kann verwendet werden, um im MRF z.B. über hydrodynamische Effekte eine Schmierung, sowie Stofftransport und Kühlung zu erreichen. Außerdem kann eine Strömung von der Dichtung weg erzielt werden und es können Druckunterschiede abgebaut werden.

Um z.B. das Spiel zwischen zwei Teilen einzustellen oder aber um Spiel aus einer Konstruktion herauszunehmen und um z.B. Fertigungstoleranzen auszugleichen kann eine Kraft bzw. eine Axialkraft und/oder eine Radialkraft eingesetzt werden, die durch einen MRF Keileffekt hervorgerufen wird.

Durch den Keil oder den Aufbau eines Keils bzw. einer MRF Schicht kann das Laufspiel von Kugel- oder Rollen- oder Nadellagern bis zu Null reduziert werden. Insbesondere mit Schrägkugellagern oder Kegelrollenlagern funktioniert das sehr gut, da hier das Spiel durch die Konstruktion voreingestellt bzw. einstellbar ist. Bei großem Spiel kann beim Aufbau des Keils ein axiales Verfahren erzwungen werden. In diesem Anwendungsfall wird der MRF-Keileffekt nicht als Kupplung oder als Bremse eingesetzt, sondern zum Einstellen des Lagerspiels.

In Weiterbildungen ist es möglich, eine Radial- oder Axialkraft z.B. eines z.B. Schrägkugellagers gegen eine Feder oder ein nachgiebiges Element wie beispielsweise Gummi wirken zu lassen. Es ist möglich, nicht nur zwischen zwei festen Begrenzungsflächen zu arbeiten, sondern auch, dass ein fester Anschlag und ein federbeaufschlagter Anschlag verwendet werden. So kann ein größerer Verstellbereich und eine geringere Federsteifigkeit erzielt werden.

Der bzw. ein MRF Keil kann durch ein Magnetfeld eines Magneten erzeugt werden. Ein Permanentmagnet kann von Hand verstellbar sein oder es ist auch möglich, den Permanentmagneten oder eine Abschirmung von Hand oder aktorisch bewegt zu verschieben oder zu verdrehen, um die Feldstärke im relevanten Bereich zu erhöhen oder zu verringern. Ein beliebiger Teil des magnetischen Kreises kann relativ zu diesem bewegt werden um das im MRF Keil wirkende Magnetfeld zu beeinflussen.

Es kann eine mechanische Fein- oder Grobjustage und damit auch ein Einstellen der Bremswirkung möglich sein. Eine solche Einstellung kann z.B. zur Kompensation von physikalischen Größen wie Temperatur, Druck, Drehzahl oder dergleichen vorgesehen sein. Möglich ist auch ein Ausgleich von Toleranzen oder Montageungenauigkeiten.

In allen Ausgestaltungen ist es bevorzugt, eine einstellbare dauerhafte Magnetfeldstärke über Remanenz vorzusehen. In bevorzugten Ausführungsformen hat ein Lager mit einer erfindungsgemäßen magnetorheologischen Übertragungsvorrichtung selbst keinen oder nur minimalen Restmagnetismus (Remanenz). Ansonsten kann es zu einer positionsabhängigen und unterschiedlich starken Gegenkraft kommen, da sich die Teile zueinander bewegen.

Das Remanenzmaterial sollte in vorteilhaften Ausgestaltungen in einem allgemeinen Bereich des Lagers angeordnet sein, der insbesondere positionsunabhängig von dem Magnetfeld durchflutet wird, so z.B. die innere Welle oder die äußere Hülle etc.

Es ist aber auch bevorzugt, den Effekt der positionsabhängigen Magnetisierung zu nutzen, indem z.B. die innere Lauffläche mit Remanenz eingesetzt wird, um beispielsweise bestimmte Rastmomente zu erzeugen. Das kann z.B. für eine haptische Rückkopplung über veränderbare Rastmomente bezüglich ihrer Stärke, des Drehwinkels oder des Endanschlags oder dergleichen erfolgen. Je nach gewünschter Einstellbarkeit müssen nicht alle Lagerkugeln ferromagnetisch sein.

Es ist auch möglich, eine magnetorheologische Übertragungsvorrichtung mit vom klassischen Lageraufbau abweichender Gestaltung vorzusehen. Die Richtung des Magnetfeldes kann z.B. wenigstens teilweise oder vollständig auch etwa parallel zur Achse ausgerichtet sein. Möglich ist auch eine wenigstens teilweise Ausrichtung parallel zur Dreh- oder Bewegungsrichtung oder in tangentialer Richtung. Möglich ist es auch, dass der gesamte magnetische Kreis nahezu oder vollständig im Inneren angeordnet ist.

Das Material des magnetorheologischen Übertragungsvorrichtung muss nicht komplett ferromagnetisch sein, je nach gewünschter Anwendung bzw. Magnetisierung kann es vorteilhaft sein, wenn einzelne Bestandteile der magnetorheologischen Übertragungsvorrichtung nicht bzw. nur teilweise ferromagnetisch sind.

Je nach Anwendung ist es auch denkbar, wenigstens einen Teil aus verschiedenen Materialien zu fertigen, um lokal unterschiedliche magnetische Eigenschaften zu erhalten.

Eine mögliche Ausführung ist ein Drehknopf mit einem integriertem Drehgeber und einer magnetorheologischen Übertragungsvorrichtung mit Keileffekt. Die Position bzw. der Drehwinkel des Drehknopfs kann über den Drehgeber bestimmt und der Drehwiderstand in einem weiten Bereich verändert werden. Dadurch kann beispielsweise ein haptisches Interface mit veränderbaren Rastmomenten und beliebig einstellbarem Endanschlag aufgebaut werden, welcher je nach gerade angewähltem Menü seine Eigenschaften ändert. Es kann ein niederes oder hohes Moment und/oder ein kleines oder großes Raster/Rippel und auch ein variables Raster - je nach zu bedienendem Menü - eingestellt werden. Der Verlauf der Momentenzu- und -abnahme kann situationsabhängig eingestellt oder variiert werden, beispielsweise als rechteck-, sinus-, sägezahnförmiger oder beliebiger Verlauf. Auch ein Anschlag kann simuliert werden. Der Anschlag kann hart sein oder einen vordefinierten oder situationsabhängigen Momentenverlauf haben.

Der Drehknopf als eine Komponente ist vorzugsweise fest mit der Welle als anderer Komponente verbunden, welche wiederum drehbar im Gehäuse gelagert ist. Die Relativbewegung bzw. Relativposition wird über einen Drehgeber erfasst, z.B. über einen magnetischen, optischen oder (über Tasten) mechanischen Inkrementalgeber. Auch ein Potentiometer mit Schleifkontakten kann eingesetzt werden, mit diesem sind aber üblicherweise nur bestimmte Drehwinkel zulässig.

Ein Dichtring ist vorteilhaft, damit die magnetorheologische Flüssigkeit im Gehäuse bleibt. Die Dichtung kann auch nur aus Permanentmagneten oder einer Kombination aus Permanentmagnet und herkömmlicher Dichtung bestehen.

Der innere Bereich, d.h. das von Dichtung und Gehäuse eingeschlossene Volumen, ist zumindest teilweise mit einer magnetorheologischen Flüssigkeit gefüllt.

Das Gehäuse ist vorzugsweise als Topf ausgebildet, d.h. einseitig geschlossen. Dadurch wird nur ein Dichtring benötigt. Eine durchgehende Welle (beidseitige Welle) ist auch denkbar.

Die Spule kann ein Magnetfeld erzeugen, wobei der magnetische Kreis über das Gehäuse, die Welle und die magnetorheologische Übertragungsvorrichtung geschlossen wird. Dadurch kann sich in der magnetorheologischen Übertragungsvorrichtung das für den Keileffekt nötige Magnetfeld aufbauen. Vorteilhafter Weise ist die Spule fest mit dem Gehäuse verbunden, was die Kabelführung erleichtert.

Der Aufbau ist robust und kann so ausgelegt sein, dass fast keine magnetischen Streufelder außerhalb des Gehäuses erzeugt werden. Denkbar sind aber viele andere Aufbauvarianten, die je nach Anwendungsfall bestimmte Vorteile haben können.

Beispielsweise kann die Spule auch außerhalb des Gehäuses angeordnet sein, wobei das Magnetfeld dann durch das Gehäuse hindurch auf die magnetorheologische Übertragungsvorrichtung wirkt. Dabei ist keine mechanische Verbindung zwischen Spule und Gehäuse nötig, das Koppeln der magnetischen Kreise reicht, um die magnetorheologische Übertragungsvorrichtung im Gehäuse zu beeinflussen. Insbesondere muss die Spule nicht dauerhaft am oder in der Nähe des Gehäuses sein und kann derart gestaltet werden, dass sie als separate Einheit vom Gehäuse entfernt werden kann. Es können auch Permanentmagnete im Magnetkreis vorhanden sein.

Der Drehknopf kann in einer bevorzugten Ausführung beispielsweise elektromagnetisch angetrieben sein und auch aktiv eine Kraft ausüben (Force Feedback), um statisch eine gewisses Gegenmoment erzeugen zu können. Bei dieser Konstruktion wird ein besseres Drehmoment zu Bauraumverhältnis erzielt als bei vielen Konstruktionen nach dem Stand der Technik. Zudem sind die Herstellkosten wegen des einfachen Aufbaus gering, da z.B. die Abrollflächen der Komponenten bei Haptikanwendungen nicht hochpräzise sein müssen und auch in der Regel keine hohen Drehzahlen und keine große Anzahl an Umdrehungen aushalten müssen. Generell hat die hier beschriebene magnetorheologische Übertragungsvorrichtung eine sehr niedere Grundreibung (OFF State). Vorzugsweise ist auch eine Batterie und ein Steuerbefehlübertragungseinheit (Funk, WLAN, Bluetooth, ANT..) in den Steller bzw. Drehknopf integriert. Der Haptikknopf kann dann überall platziert werden und braucht keinen leitungsgebundenen Steuer- bzw. Stromanschluss. Das MRF Keilprinzip braucht in Relation zum Drehmoment sehr wenig Strom (Leistung). Deshalb ist es auch für den Batteriebetrieb oder zur drahtlosen Energieversorgung gut geeignet. Dabei können sowohl die benötigte Energie als auch Steuerbefehle und beispielsweise auch Messwerte von Sensoren wie Drehwinkel drahtlos übertragen werden.

Eine bevorzugte Ausführung kommt ohne Batterie aus und erhält die für die Funktion notwendige Energie mittels induktiver Kopplung. Besonders bevorzugt sind auch Ausführungen, die die zum Betrieb nötige Energie direkt aus der Umwelt beziehen und lokal zwischenspeichern (Energy Harvesting). Dabei sind zur Energiewandlung thermoelektrische Generatoren, Solarzellen, Elemente die Vibrationsenergie in elektrische Energie wandeln und Andere sowie entsprechende lokale Energiespeicher möglich. Denkbar ist auch, die Bewegung der magnetorheologischen Übertragungsvorrichtung selbst zur Energieerzeugung zu verwenden.

Wenn die erfindungsgemäße magnetorheologische Übertragungsvorrichtung wenigstens teilweise über einen Dauermagneten mit einem magnetischen Magnetfeld beaufschlagt wird, dessen Magnetisierung durch wenigstens einen magnetischen Impuls wenigstens einer elektrischen Spule dauerhaft verändert wird ergeben sich einige Vorteile. In bestimmten Fällen kann z.B. durch die Ausnutzung der Remanenz und des Impulsbetriebs einer Spule, die nicht immer bestromt werden muss, ein Gewichts- und Raumvorteil erzielt werden. Die Drähte der Spule können dünner und leichter dimensioniert werden, weil sie jeweils nur für eine kurze Betriebszeit bestromt werden. Das kann Vorteile bei Gewicht, Strombedarf, Platzbedarf und Kosten ergeben.

Deshalb kann es in bestimmten Anwendungen vorteilhaft sein, dass durch den Impulsbetrieb der elektrischen Spule diese deutlich kleiner ausgeführt werden kann als wenn sie auf 100% Einschaltdauer ausgelegt werden muss. Die Erwärmung der Spule spielt im Impulsbetrieb üblicherweise keine Rolle, da kurzzeitige Verlustleistungsspitzen von der eigenen Wärmekapazität der Spule und der die Spule umgebenden Bauteile abgepuffert werden. Dadurch können sehr hohe Stromdichten in den Windungen toleriert werden bzw. können dünnere Leitungen verwendet werden, solange die mittlere Verlustleistung über längere Zeiträume akzeptabel bleibt.

Üblicherweise kann bei einer kleineren Spule auch der die Spule umgebende magnetische Kreis kleiner ausfallen, weshalb verhältnismäßig viel Bauraum, Material, Gewicht und Kosten eingespart werden können. Dabei steigt lediglich der Energieaufwand für einen einzelnen Puls, was aber je nach Anwendung sehr wohl toleriert werden kann. Insgesamt kann dennoch viel Energie im Vergleich zu einer dauerhaft bestromten Spule eingespart werden.

In allen Ausgestaltungen kann es möglich sein, die Stromversorgung leitungslos zu realisieren. Die Stromversorgung beispielsweise von der Stromquelle zur Leistungselektronik bzw. von der Leistungselektronik zu der Spule kann über eine elektrische, magnetische oder elektromagnetische Kopplung wie beispielsweise eine Funkstrecke erfolgen. Bei der Anwendung in einem Fahrrad kann die Stromversorgung von außen über z. B. eine Docking Station erfolgen. Möglich ist auch die Energieversorgung über eine Energiequelle an z.B. einem Fahrrad auf alle Verbraucher (Gabel, Dämpfer Hinten, Display). Analog kann auch die Stromversorgung bei einem Skischuh, Ski, Mobiltelefon oder zu den Sensoren erfolgen.

Eine Energieversorgung über Funk kann möglicherweise einen schlechteren Wirkungsgrad als eine herkömmliche Verkabelung aufweisen. Außerdem kann die Energieübertragung und deren Reichweite begrenzt sein. Je nach Anwendungsfall stören solche Nachteile aber nicht. Vorteilhaft ist, dass kein Verschleiß der Kontakte auftritt. Die Energieübertragung ist üblicherweise verpolsicher und kurzschlussfest, da sekundärseitig nur eine begrenzte Leistung vorliegt. Weiterhin ist kein Kabelbruch möglich und die Vorrichtung ist insgesamt beweglicher.

Bei solchen Ausgestaltungen ist es aber vorteilhaft, die Energie für zumindest einen Impuls in einem Kondensator oder Energiespeicher zwischenzuspeichern. Dadurch kann die Energieversorgung des Systems eine kleinere Leistung haben, da kurzzeitige Leistungsspitzen eines Impulses vom Kondensator abgefangen werden. Zudem kann auch eine unstetige oder impulsförmige Energieversorgung eingesetzt werden.

Eine mögliche Ausbaustufe der vorliegenden Erfindung ist ein vollkommen autarkes System, welches kabellos mit Energie versorgt wird. Vorstellbar ist beispielsweise die Anwendung an einem Fahrrad, wobei durch zumindest einen kleinen Magneten an einem Reifen das System mit Energie versorgt wird.

Generell können so beliebige "Energy Harvesting"-Einheiten zur Energieversorgung verwendet werden, beispielsweise Solarzellen, thermoelektrische Generatoren oder Piezo-Kristalle. Auch Elemente, welche Vibrationen in Energie wandeln, können so sehr vorteilhaft zur Versorgung eingesetzt werden.

Denkbar ist auch eine Ausführung ähnlich wie bei einer elektrischen Zahnbürste, bei der die Energieversorgung durch induktive Kopplung erfolgt. Dabei kann beispielsweise der Akku induktiv geladen werden, ohne dass beschädigte Kabel, korrodierte oder verschmutzte Kontakte den Ladevorgang behindern. Über längere Strecken kann Energie über magnetische Resonanz übertragen werden.

Die Stromversorgung des Remanenzimpulses kann via Induktion wie bei elektrischen Zahnbürsten erfolgen. Die Kombination des MRF Keilprinzips mit Remanenz ist besonders stromsparend und vorteilhaft.

Es kann auch ein Lautsprecher bzw. eine Geräuscherzeugungseinheit integriert oder zugeordnet sein. Das ist vorteilhaft, da der Drehknopf als MRF Keilknopf an sich mechanisch geräuschlos ist. Sowohl das Drehen ohne als auch mit Raster oder/und die virtuellen Anschlägen sind an sich geräuschlos. Das Erzeugen des MRF Keils zu einer Drehmomenterhöhung oder zur Erzeugung eines Rasters ist ebenso an sich geräuschlos. Mittels der Geräuschquelle wie einem Lautsprecher oder einem Piezolautsprecher kann z.B. dem virtuellen Raster ein Klicken bei jeder Rastposition zugeordnet werden. Die Art, Lautstärke und Dauer des Geräusches kann individuell zugeordnet werden, aber bei Benutzerwunsch auch verändert oder abgeschaltet werden.

Somit ist das Drehmoment, der Raster, die Anschläge und das Geräusch programmierbar bzw. adaptiv. Die Geräusche können auch über externe Lautsprecher wie z.B. Standardlautsprecher im Auto oder die Lautsprecher der Hi-Fi Anlage im Haus erzeugt werden.

Der Haptikknopf kann somit praktisch das Mausrad einer Computermaus ersetzen. Bei dem Raster kann nicht nur der Winkelabstand des Rasters einstellbar sein, sondern auch dessen Verlaufsform, Stärke usw. Damit kann quasi eine Rasterkennlinie vorgeben werden.

Der Haptikdrehknopf kann auch auf einer Bedienfläche oder an einem Bildschirm montiert sein. Damit man das Display nicht für die Befestigung des Knopfes herausnehmen muss, kann dieser aus einem Oberteil auf dem Display und einem Unterteil unterhalb des Displays bestehen. Vorzugsweise ist eine Datenübertragung über z.B. Induktion oder dergleichen vorgesehen. Dadurch kann das Display als eine Fläche billiger hergestellt werden.

Es ist auch möglich, dass ein MRF Haptikknopf sich auch drücken lässt. Dabei kann das Drücken auch durch ein MRF wirken, dessen Eigenschaften über ein Magnetfeld veränderbar sind.

Der Bildschirm zeigt die einzustellenden Informationen an, welche sich je nach Anwendung ändern. Die Funktion des Haptikknopfes passt sich daran an. Einmal wird mittels eines Rasters verstellt (z.B. Einstellen der Lautstärke; auf dem Display erscheint eine Lautstärkenskala, welche auch eine logarithmische Skalierung haben kann).

Ein anderes Mal kann zwischen zwei Positionen ohne Raster aber mit variablem Moment verstellt werden, so z.B. zwischen der Uhrstellung 8:00 und der Uhrstellung 16:00, wobei vor der Endposition jeweils ein zunehmendem Moment vorgesehen sein kann. Das Raster kann auch zum Anfahren definierter Positionen dienen, wenn z.B. nach einer Nameneingabe gefragt wird.

Das Display kann auch als Touchscreen ausgeführt sein. Dadurch können Menüpunkt rasch gewählt und mittels des Drehstellers Feineinstellungen gemacht werden. Z.B. ist es bei Autos nicht gewünscht, die Lautstärkenregelung des Radios über Touchscreen zu machen, da der Fahrer sonst immer lange hinschauen müsste, was und wo er gerade verstellt, was ihn ablenkt. Den Drehsteller findet er auch mit einem kurzen Blick oder ohne Hinsehen.

Auch z.B. beim Fahrradfahren ist das Verstellen mit einem mechanischen Steller einfacher und sicherer als über ein Touchdisplay. Dies insbesondere auch dann, wenn der Radfahrer z.B. Handschuhe an hat, wodurch die Bedienung eines Touchdisplay schwer oder sogar unmöglich ist.

Es ist auch eine Kombination von einem Display oder Touchdisplay und einem mechanischem Drehsteller mit variablem Moment/Raster möglich. Solche Eingabegeräte können auch außerhalb des Kraftfahrzeuges vorteilhaft sein, so z.B. bei Steuerungen für Industrieanlagen, Fernbedienungen für z.B. Fernseher oder Funkfahrzeuge wie Spielzeughubschrauber, sowie an PC und Spielkonsolen, Steuerkonsolen für militärische Anwendungen (Drohnenflugzeuge, Raketen).

Möglich ist es auch, dass ein Haptikdrehknopf mit einer Anzeige die jetzige Computermaus ersetzt.

Es ist möglich, dass der Drehknopf bzw. das Stellglied in normalen Zustand versenkt sein kann und nur bei Bedarf ausgefahren wird.

Es ist auch möglich, eine solche Baueinheit als Schieberegler auszuführen, insbesondere in Kombination mit einer linearen MRF Keileinheit.

Es ist auch möglich, eine magnetorheologische Übertragungsvorrichtung mit einem oder mehreren Polen und einer oder mehreren Erhebungen auszustatten. In allen Ausgestaltungen ist es möglich, dass zwischen den zwei Komponenten der magnetorheologischen Übertragungsvorrichtung Erhebungen oder dergleichen vorgesehen sind, die beispielsweise von der einen Komponente in Richtung auf die andere Komponente hervorstehen.

Sowohl bei einer rotativen als auch bei einer linearen Beweglichkeit der beiden Komponenten zueinander ist eine solche Ausgestaltung möglich und bevorzugt.

Dabei kann nur eine Erhebung vorgesehen sein oder es können mehrere vorgesehen sein. Möglich ist es, dass an wenigstens einer Erhebung eine Kugel oder eine Rolle oder ein sonstiger Drehkörper angeordnet ist, der von der Erhebung wenigstens teilweise aufgenommen wird.

Wenn an der einen Komponente Erhebungen vorgesehen sind, ist es bevorzugt, dass an der anderen Komponente wenigstens ein Pol bzw. wenigstens eine Magnetisierungseinheit oder wenigstens ein Magnet oder einer Spule vorgesehen ist. Die Anzahl der Magnetisierungseinheiten bzw. Pole kann 1 betragen oder auch größer sein.

Die Form der Erhebungen kann grundsätzlich beliebig und z.B. halbrund, spitz oder stumpf sein. Der Aufnahmebereich von Drehkörpern ist vorzugsweise entsprechend abgerundet ausgebildet.

Eine oder mehrere Magnetisierungseinheiten bzw. Pole können als Elektrospule plus Kern, oder als Permanentmagnet ausgebildet sein oder aus Remanenzmaterial oder einen Kombination aus diesen bestehen.

Die Abstände zwischen einzelnen Erhebungen und/oder Magnetisierungseinheiten sind vorzugsweise etwa gleichbleibend, können aber auch beliebig sein.

Die Tiefe, d.h. die radiale Erstreckung oder die axiale Erstreckung einzelner Erhebungen oder Magnetisierungseinheiten zu anderen kann unterschiedlich sein.

Auch die an den einzelnen Magnetisierungseinheiten anliegende oder wirkende Feldstärke kann insbesondere auch zur gleichen Zeit variieren.

Die Drehzahl der Drehkörper muss nicht gleich der Abrolldrehzahl sein, sie kann auch davon abweichen, z.B. durch Unter- oder Übersetzungen. Das Innenteil, welches durch die Erhebungen z. B. sternförmig ausgebildet ist, kann zum Außenteil außermittig gelagert sein.

Eine Anwendung einer solchen magnetorheologischen Übertragungsvorrichtung kann z.B. als Haptikknopf mit Raster oder bei Möbel- und Schubladenführungen mit Positionen erfolgen.

Der Magnet bzw. jede Magnetisierungseinheit oder das Innenteil und/oder das Außenteil können auch aus Remanenzmaterial bestehen.

Die Vorrichtung kann bei Einsatz an einer Lenksäule dazu dienen, die Lenkung um die Mittellage herum zu bremsen, damit z.B. bei einer von Hause aus leichtgängigen Servolenkung die Lenkung schwerer geht, was z.B. bei einer Geradeausfahrt auf einer Autobahn vorteilhaft ist. Eine magnetorheologische Übertragungsvorrichtung kann solche leichtgängigen Lenkungen bremsen und durch ein auf die jeweilige Situation bzw. den Anwender angepasstes Moment den Fahrkomfort erhöhen.

Möglich ist es auch, andere Lenkvorgänge sicher zu gestalten, indem z.B. Endanschläge mit einer magnetorheologischen Übertragungsvorrichtung realisiert werden. Damit werden auch bei hydraulischen Lenkungen Funktionen ermöglicht, die zuvor nur über elektronische Lenkungen realisiert werden konnten.

Da magnetorheologische Fluide bei Anlegung eines Magnetfeldes sehr schnell verketten, kann es im Normalzustand z.B. beim Autofahren ausreichen, wenn das magnetische Feld abgeschaltet ist. Es reicht in der Regel völlig aus, erst einzuschalten, wenn eine erste Drehwinkeländerung eingeleitet wird. Dadurch kann erheblich Energie eingespart werden.

Alternativ dazu kann mit Remanenz ein Grundmoment realisiert werden. Wenn eine Drehwinkeländerung registriert wird, kann ein dynamisches Magnetfeld aufgebaut werden, welches auch pulsieren kann, um ein virtuelles Raster zu erzeugen.

In allen Ausgestaltungen ist es möglich, eine z.B. adaptive Türbremse zu realisieren. Dazu kann z.B. bei einem KFZ beim Einparken eine Parklücke vermessen werden. Aus den Daten kann der Abstand zum benachbarten KFZ berechnet werden. Daraus wiederum kann der maximal öffenbare Türwinkel errechnet werden und bei Erreichen oder schon zuvor der Öffnungsvorgang entsprechend abgebremst werden.

Dazu kann der Sensor oder es können die Sensoren zum Messen des Abstandes von Fahrzeug beim Parken verwendet werden, sodass separate Sensoren nicht benötigt werden. Möglich ist es auch, die Steuerung so vorzunehmen, dass die Tür zunächst leicht aufgeht und dass dann ein Raster kommt, das immer feiner wird. Damit wäre praktisch eine Haptikanzeige für Türöffner realisiert, die anzeigt, wenn man in die Nähe des Anschlages kommt.

Möglich ist es auch, Türen, Fenster oder dergleichen in bestimmten Winkel auf zu halten. Das kann z.B. bei Kraftfahrzeugen (KFZ) oder auch bei Möbeln realisiert werden.

In Ausgestaltungen, in denen die Remanenz genutzt wird, kann das Magnetfeld zum Ummagnetisieren von außen aufgeprägt werden. Zum Ummagnetisieren kann eine entsprechende Spule verwendet werden, die durch z.B. einen Zylinder wirkt.

Bei dem erfindungsgemäßen Verfahren wird die Kopplungsintensität wenigstens zweier koppelbarer Komponenten mit einer magnetorheologischen Übertragungsvorrichtung beeinflusst, wobei die Kopplungsintensität in wenigstens einem Kanal beeinflusst wird, der ein durch ein Magnetfeld beeinflussbares magnetorheologisches Medium mit magnetisch polarisierbaren Partikeln enthält, und wobei mit wenigstens einer Magnetfelderzeugungseinrichtung wenigstens ein Magnetfeld in dem Kanal erzeugt wird, um mit dem Magnetfeld das magnetorheologische Medium in dem Kanal zu beeinflussen. Dabei ist in dem Kanal wenigstens ein Drehkörper vorgesehen und ein freier Abstand zwischen dem Drehkörper und der Komponente ist größer als der zehnfache Durchmesser des typischen mittleren magnetisch polarisierbaren Partikels. Zwischen dem Drehkörper und wenigstens einer Komponente ist wenigstens ein spitzwinkliger und das magnetorheologische Medium enthaltender Bereich vorgesehen. Der Kanal wird wenigstens zeitweise und wenigstens teilweise mit dem Magnetfeld der Magnetfelderzeugungseinrichtung beaufschlagt, um die Partikel wahlweise zu verketten und/oder mit dem Drehkörper zu verkeilen oder freizugeben.

Weitere Vorteile und Merkmale der vorliegenden Erfindung ergeben sich aus den Ausführungsbeispielen, die mit Bezug auf die beiliegenden Figuren im Folgenden erläutert werden.

In den Figuren zeigen:
- Fig. 1: eine stark schematische Ansicht einer erfindungsgemäßen magnetorheologischen Übertragungsvorrichtung im Querschnitt;
- Fig. 2: eine als Kupplung ausgeführte erfindungsgemäße magnetorheologische Übertragungsvorrichtung;
- Fig. 3: eine als Bremse ausgeführte magnetorheologische Übertragungsvorrichtung;
- Fig. 4: eine magnetorheologische Übertragungsvorrichtung zur Beeinflussung von Linearbewegungen;
- Fig. 5: einen vergrößerten Querschnitt der Vorrichtung nach Fig. 4;
- Fig. 6: eine magnetorheologische Übertragungsvorrichtung mit einer Kühleinrichtung;
- Fig. 7: eine weitere magnetorheologische Übertragungsvorrichtung zur Beeinflussung von Linearbewegungen;
- Fig. 8: ein Querschnitt aus Fig. 7;
- Fig. 9: eine Prothese mit erfindungsgemäßen magnetorheologischen Übertragungsvorrichtungen;
- Fig. 10: eine stark schematische Ansicht einer Kupplung;
- Fig. 11: einen Schuh mit einer erfindungsgemäßen magnetorheologischen Übertragungsvorrichtung,
- Fig. 12: einen Drehknopf mit einer erfindungsgemäßen magnetorheologischen Übertragungsvorrichtung;
- Fig. 13: den Drehknopf nach Fig. 12 mit einer Anzeige;
- Fig. 14: einen Joystick mit einer erfindungsgemäßen magnetorheologischen Übertragungsvorrichtung;
- Fig. 15: einen weiteren Drehknopf in einer schematischen Vorderansicht;
- Fig. 16: einen anderen Drehknopf in einer schematischen perspektivischen Ansicht;
- Fig. 17: einen weitere magnetorheologische Übertragungsvorrichtung in einer geschnittenen Ansicht;
- Fig. 18: einen Kniehebel als magnetorheologischer Übertragungsvorrichtung in einer geschnittenen Ansicht;
- Fig. 19: den Kniehebel nach Fig. 18 in einer Seitenansicht;
- Fig. 20: noch eine magnetorheologische Übertragungsvorrichtung in einer Vorderansicht;
- Fig. 21: die magnetorheologische Übertragungsvorrichtung in einer geschnittenen Ansicht;
- Fig. 22: noch eine weitere magnetorheologische Übertragungsvorrichtung in einer geschnittenen Ansicht; und
- Fig. 23: die Polscheiben der magnetorheologischen Übertragungsvorrichtung nach Fig. 22.

Mit Bezug auf die beiliegenden Figuren werden im Folgenden Ausführungsbeispiele von erfindungsgemäßen magnetorheologischen Übertragungsvorrichtungen 1 erläutert, wobei gleiche oder ähnliche Teile mit den gleichen Bezugszeichen versehen werden.

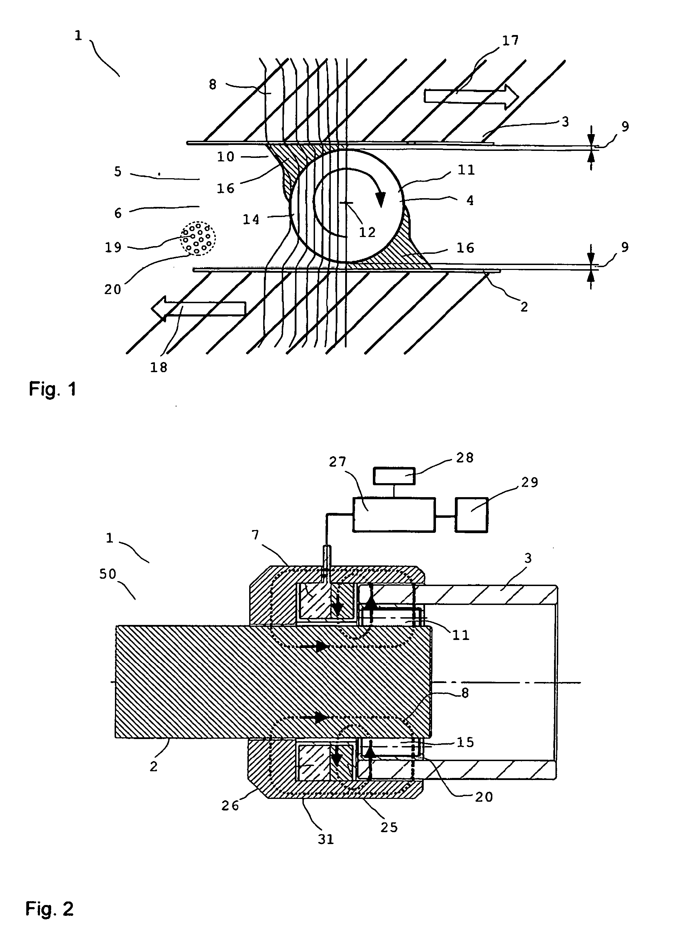

Figur 1 zeigt eine stark schematische Querschnittsansicht einer erfindungsgemäßen magnetorheologischen Übertragungsvorrichtung 1 zur Beeinflussung der Kraftübertragung zwischen zwei Komponenten 2 und 3. Dabei ist zwischen den zwei Komponenten 2 und 3 in Fig. 1 ein Drehkörper 11 als separates Teil 4 vorgesehen. Der Drehkörper 11 ist hier als Kugel 14 ausgebildet. Möglich ist es aber ebenso, Drehkörper 11 als Zylinder oder Ellipsoide, Rollen oder sonstige rotierbare Drehkörper auszubilden. Auch im eigentlichen Sinn nicht rotationssymmetrische Drehkörper wie beispielsweise ein Zahnrad 34 oder Drehkörper 11 mit einer bestimmten Oberflächenstruktur können als Drehkörper verwendet werden. Die Drehkörper 11 werden nicht zur Lagerung gegenüber einander eingesetzt, sondern zur Übertragung von Drehmoment.

Zwischen den Komponenten 2 und 3 der magnetorheologischen Übertragungsvorrichtung 1 ist ein Kanal 5 vorgesehen, der hier mit einem Medium 6 gefüllt ist. Das Medium ist hier ein magnetorheologisches Fluid 20, welches beispielsweise als Trägerflüssigkeit ein Öl umfasst, in dem ferromagnetische Partikel 19 vorhanden sind. Glykol, Fett, dickflüssige Stoffe können auch als Trägermedium verwendet werden, ohne darauf beschränkt zu sein. Das Trägermedium kann auch gasförmig sein bzw. es kann auf das Trägermedium verzichtet werden (Vakuum). In diesem Fall werden lediglich durch das Magnetfeld beeinflussbare Partikel in den Kanal gefüllt.

Die ferromagnetischen Partikel 19 sind vorzugsweise Carbonyleisenpulver, wobei die Größenverteilung der Partikel vom konkreten Einsatzfall abhängt. Konkret bevorzugt ist eine Verteilung Partikelgröße zwischen ein und zehn Mikrometern, wobei aber auch größere Partikel von zwanzig, dreißig, vierzig und fünfzig Mikrometer möglich sind. Je nach Anwendungsfall kann die Partikelgröße aus deutlich größer werden und sogar in den Millimeterbereich vordringen (Partikelkugeln). Die Partikel können auch eine spezielle Beschichtung/Mantel (Titanbeschichtung, Keramik-, Karbonmantel etc.) aufweisen, damit sie die je nach Anwendungsfall auftretenden hohen Druckbelastungen besser aushalten. Die MR-Partikel können für diesen Anwendungsfall nicht nur aus Carbonyleisenpulver (Reineisen), sondern z.B. auch aus speziellem Eisen (härterem Stahl) hergestellt werden.

Der Drehkörper 11 wird durch die Relativbewegung 17 der beiden Komponenten 2 und 3 in Rotation um seine Drehachse 12 versetzt und läuft praktisch auf der Oberfläche der Komponente 3 ab. Gleichzeitig läuft der Drehkörper 11 auf der Oberfläche der anderen Komponente 2, sodass dort eine Relativgeschwindigkeit 18 vorliegt.

Genau genommen hat der Drehkörper 11 keinen direkten Kontakt zur Oberfläche der Komponente 2 und/oder 3 und wälzt sich deshalb nicht direkt darauf ab. Der freie Abstand 9 von dem Drehkörper 11 zu einer der Oberflächen der Komponente 2 oder 3 beträgt z.B. 140 µm. In einer konkreten Ausgestaltung mit Partikelgrößen zwischen 1 µm und 10 µm liegt der freie Abstand insbesondere zwischen 75 µm und 300 µm und besonders bevorzugt zwischen 100 µm und 200 µm.

Der freie Abstand beträgt insbesondere wenigstens das zehnfache des Durchmessers eines typischen mittleren Partikeldurchmessers. Vorzugsweise beträgt der freie Abstand wenigstens das zehnfache einesgrößten typischen Partikels. Durch den fehlenden direkten Kontakt ergibt sich eine sehr geringe(s) Grundreibung/-kraft/-moment beim relativen Bewegen der Komponenten 2 und 3 zu einander.

Wird die magnetorheologische Übertragungsvorrichtung 1 mit einem Magnetfeld beaufschlagt, bilden sich die Feldlinien abhängig vom Abstand zwischen den Drehkörpern 11 und den Komponenten 2, 3 aus. Der Drehkörper besteht aus einem ferromagnetischen Material und z.B. hier aus ST 37. Der Stahltyp ST 37 hat eine magnetische Permeabilität µr von etwa 2000. Die Feldlinien treten durch den Drehkörper hindurch und konzentrieren sich in dem Drehkörper. An der hier radialen Ein- und Austrittsfläche der Feldlinien an dem Drehkörper herrscht eine hohe Flußdichte in dem Kanal 5. Das dort inhomogene und starke Feld führt zu einer lokalen und starken Vernetzung der magnetisch polarisierbaren Partikel 19. Durch die Drehbewegung des Drehkörpers 11 in Richtung auf den sich bildenden Keil in dem magnetorheologischen Fluid wird die Wirkung stark erhöht und das mögliche Brems- oder Kupplungsmoment wird extrem vergrößert weit über den Betrag hinaus, der normalerweise in dem magnetorheologischen Fluid erzeugbar ist. Vorzugsweise bestehen Drehkörper 11 und Komponente 2, 3 zumindest teilweise aus ferromagnetischem Material, weshalb die magnetische Flussdichte umso höher wird, je kleiner der Abstand zwischen Drehkörper 11 und Komponente 2, 3 ist. Dadurch bildet sich ein im Wesentlichen keilförmiger Bereich 16 im Medium aus, in welchem der Gradient des Magnetfelds zum spitzen Winkel bei der Kontaktstelle/ dem Bereich des geringsten Abstands hin stark zunimmt.

Trotz Abstand zwischen Drehkörper 11 und Komponente 2, 3 kann durch die Relativgeschwindigkeit der Oberflächen zueinander der Drehkörper 11 in eine Drehbewegung versetzt werden. Die Drehbewegung ist ohne und auch mit einem wirkenden Magnetfeld 8 möglich.

Wenn die magnetorheologische Übertragungsvorrichtung 1 einem Magnetfeld 8 einer hier in Figur 1 nicht dargestellten Magnetfelderzeugungseinrichtung 7 ausgesetzt ist, verketten sich die einzelnen Partikeln 19 des magnetorheologischen Fluides 20 entlang der Feldlinien des Magnetfeldes 8. Zu beachten ist, dass die in Figur 1 eingezeichneten Vektoren den für die Beeinflussung des MRF 20 relevanten Bereich der Feldlinien nur grob schematisch darstellen. Die Feldlinien treten im Wesentlichen normal auf die Oberflächen der ferromagnetischen Bauteile in den Kanal 5 ein und müssen vor allem im spitzwinkligen Bereich 10 nicht geradlinig verlaufen.

Gleichzeitig wird auf dem Umfang des Drehkörpers 11 etwas Material von dem magnetorheologischen Fluid 20 mit in Rotation versetzt, sodass sich ein spitzwinkliger Bereich 10 zwischen der Komponente 3 und dem Drehkörper 11 ausbildet. Auf der anderen Seite entsteht ein gleicher spitzwinkliger Bereich 10 zwischen dem Drehkörper 11 und der Komponente 2. Die spitzwinkligen Bereiche 10 können beispielsweise bei zylinderförmig ausgestalteten Drehkörpern 11 eine Keilform 16 aufweisen. Durch die Keilform 16 bedingt wird die weitere Rotation des Drehkörpers 11 behindert, sodass die Wirkung des Magnetfeldes auf das magnetorheologische Fluid verstärkt wird, da sich durch das wirkende Magnetfeld innerhalb des spitzwinkligen Bereiches 10 ein stärkerer Zusammenhalt des dortigen Mediums 6 ergibt. Dadurch wird die Wirkung des magnetorheologischen Fluids im angesammelten Haufen verstärkt (die Kettenbildung im Fluid und damit der Zusammenhalt bzw. die Viskosität), was die weitere Rotation bzw. Bewegung des Drehkörpers 11 erschwert.

Durch die Keilform 16 können wesentlich größere Kräfte oder Momente übertragen werden als es mit einem vergleichbaren Aufbau möglich wäre, der nur die Scherbewegung ohne Keileffekt nützt.

Die direkt durch das angelegte Magnetfeld übertragbaren Kräfte stellen nur einen kleinen Teil der durch die Vorrichtung übertragbaren Kräfte dar. Durch das Magnetfeld lässt sich die Keilbildung und somit die mechanische Kraftverstärkung steuern. Die mechanische Verstärkung des magnetorheologischen Effekts kann soweit gehen, dass eine Kraftübertragung auch nach Abschalten eines angelegten Magnetfeldes möglich ist, wenn die Partikel verkeilt wurden.

Es hat sich herausgestellt, dass durch die Keilwirkung der spitzwinkligen Bereiche 10 eine erheblich größere Wirkung eines Magnetfeldes 8 einer bestimmten Stärke erzielt wird. Dabei kann die Wirkung um ein Vielfaches verstärkt werden. In einem konkreten Fall wurde eine etwa zehnmal so starke Beeinflussung der Relativgeschwindigkeit zweier Komponenten 2 und 3 zueinander wie beim Stand der Technik bei MRF Kupplungen beobachtet. Die mögliche Verstärkung hängt von unterschiedlichen Faktoren ab. Gegebenenfalls kann sie durch eine größere Oberflächenrauhigkeit der Drehkörper 11 noch verstärkt werden. Möglich ist es auch, dass auf der Außenoberfläche der Drehkörper 11 nach außen ragende Vorsprünge vorgesehen sind, die zu einer noch stärkeren Keilbildung führen können.

Die Keilwirkung bzw. der Keileffekt verteilt sich flächig auf den Drehkörper 11 und die Komponenten 2 oder 3.

Figur 2 zeigt eine Kupplung 50 mit einer erfindungsgemäßen magnetorheologische Übertragungsvorrichtung 1, wobei die Komponenten 2 und 3 hier als rotierende Teile ausgebildet sind. An einer feststehenden Komponente 31 ist die Magnetfelderzeugungseinrichtung 7 vorgesehen, die hier eine Spule 26 und einen Dauermagneten 25 umfasst. Die Spule 26 ist mit einer Steuer-/oder Regeleinrichtung 27 verbunden. Mit dem Dauermagneten 25 kann ein dauerhaftes Magnetfeld 8 aufgebracht werden, wobei das im Kanal wirkende Magnetfeld durch Aktivierung der elektrischen Spule 26 modulierbar ist. So kann das wirkende Magnetfeld abgesenkt oder verstärkt werden.

Hier im Ausführungsbeispiel ist es bevorzugt, dass die Spule 26 zur Abgabe starker magnetischer Impulse ausgebildet ist, mit denen der Dauermagnet 25 dauerhaft veränderbar ist. Durch kurzzeitige Impulse im Bereich von 0,1 bis 1000 Millisekunden kann die Magnetisierung des Dauermagneten 25 gezielt zwischen null und seiner Remanenz verändert werden. Im Anschluss an den Impuls bleibt die magnetische Feldstärke des Dauermagneten 25 praktisch beliebig lang unverändert erhalten. Durch geeignete Modulierung der Impulse kann so beliebig häufig die wirkende Feldstärke des Dauermagneten 25 eingestellt werden, sodass auch ohne dauerhafte Energieversorgung eine bestimmte Feldstärke erzeugbar ist.

Um auch ohne dauerhaften Stromanschluss die Stärke des Magnetfeldes des Dauermagneten 25 variieren zu können, kann ein Energiespeicher 28 vorgesehen sein, der beispielsweise als Kondensator ausgeführt ist und die Energie für wenigstens einen Impuls bereit hält. Zur gezielten Regelung der Feldstärke des Dauermagneten 25 kann mindestens ein Sensor 29 vorgesehen sein, der beispielsweise die wirkende Magnetfeldstärke misst. Möglich ist es auch, dass der Sensor weitere Daten, wie das Drehmoment, die Drehzahl, die Relativgeschwindigkeit, den Drehwinkel der beiden Komponenten 2 und 3 zueinander oder die herrschende Temperatur oder dergleichen erfasst. Bei Bedarf können entsprechende Schritte eingeleitet werden, wenn beispielsweise die zulässige Temperatur der magnetorheologischen Übertragungsvorrichtung 1 überschritten wird.

Vorstellbar ist auch die Verwendung einer mechanischen Einstellvorrichtung, bei der die Feldstärke im Kanal beispielsweise durch Bewegen des Magneten, Polschuhen oder Abschirmblechen veränderte werden kann. Diese mechanische Einstellung kann auch in Kombination mit einem Bowdenzug und/oder einer elektrischen Verstellung verwendet werden, zum Beispiel wenn der Permanentmagnet eine Grundkraft als Arbeitspunkt einstellt und eine Steuerung mittels Spule die Kraft um diesen Arbeitspunkt verändern kann.

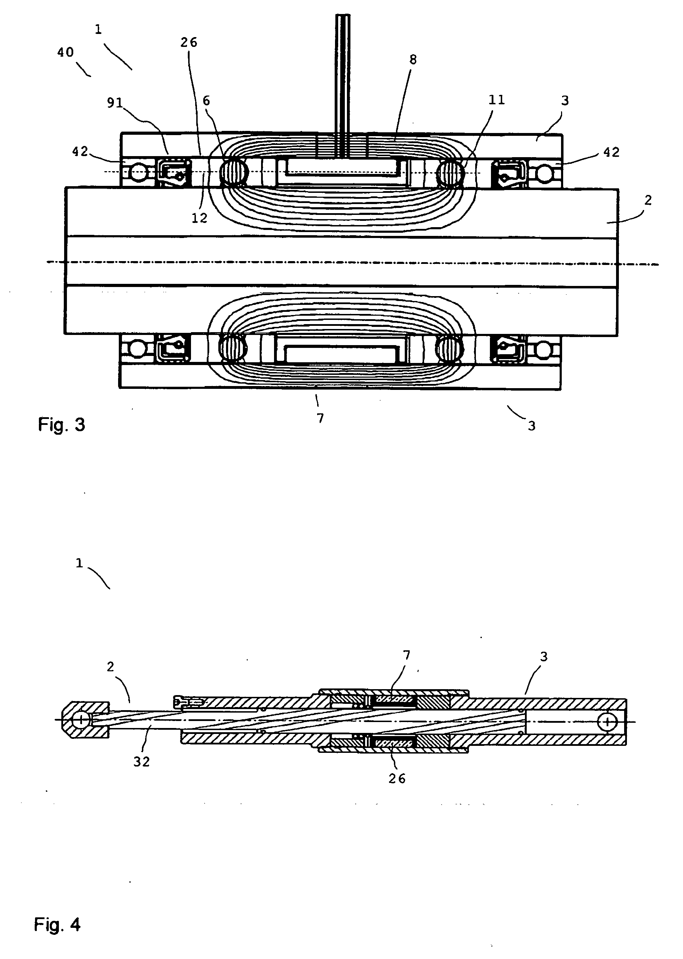

Figur 3 zeigt eine erfindungsgemäße magnetorheologische Übertragungsvorrichtung 1, die als Bremse 40 ausgebildet ist. Dabei weist die magnetorheologische Übertragungsvorrichtung 1 eine als Welle ausgebildete Komponente 2 auf, deren Rotationsbewegung relativ zu der feststehenden Komponente 3 beeinflussbar ist. Zwischen der feststehenden Komponente 3 und der drehbaren Komponente 2 sind Lager 42 zur drehbaren Lagerung der Komponenten 2, 3. vorgesehen. Die Drehkörper 11 zwischen den Komponenten 2 und 3 sind hier als Kugeln 14 ausgeführt und sind von dem Medium 6 bzw. dem magnetorheologischen Fluid 20 umgeben. Zum Schutz des Lagers 42 und um Austritt von magnetorheologischem Fluid zu vermeiden, sind Dichtungen 91 zwischen den Drehkörpern 11 und den Lagern 42 vorgesehen.

Eine beispielsweise als Spule ausgeführte Magnetfelderzeugungseinrichtung 7 dient zur gezielten Steuerung eines Magnetfeldes 8, welches sich auch durch die Drehkörper 11 erstreckt und dort im Wesentlichen quer und hier sogar senkrecht zu der Relativbewegung der beiden Komponenten 2 und 3 zueinander ausgerichtet ist. Durch die Rotationsbewegung der Drehkörper 11 wird bei eingeschaltetem Magnetfeld 8 eine Verkettung der Partikel 19 in dem magnetorheologischen Fluid 20 bewirkt, wodurch an jedem einzelnen Drehkörper 11 der spitzwinklige Bereiche 10 entstehen, die eine weitere Rotation der Komponente 2 relativ zur Komponente 3 erheblich behindern. Die Wirkung des magnetorheologischen Fluides wird dadurch erheblich verstärkt.

Das MRF Keilgehäuse kann über eine (bestehende) Antriebswelle geschoben werden, diese wird dann abhängig vom wirkenden Magnetfeld 8 gebremst, wobei sich der MRF Keil zwischen der Wellenoberfläche und den Drehkörpern 11 einstellt. Damit ergibt sich ein sehr einfacher Aufbau. Normale Bremsen oder Kupplungen benötigen hierfür regelmäßig eine Scheibe oder andere Flanschteile und haben axial gesehen eine fixe Wellenposition. Bei einem MRF Keilgehäuse gemäß der Erfindung kann die Welle axial verschoben werden, ohne dass sich das auf den Keileffekt auswirkt. Ein separater Laufring muss auf der als Welle dienenden Komponente 2 nicht aufgebracht werden.

Figur 4 zeigt eine magnetorheologische Übertragungsvorrichtung 1 zur Beeinflussung der Linearbewegungen zweier Komponenten 2 und 3 relativ zueinander. Dabei umfasst die magnetorheologische Übertragungsvorrichtung 1 eine Stange 32, die in die Komponente 3 eintaucht und dort relativ zur Komponente 3 verschiebbar vorgesehen ist. Die magnetorheologische Übertragungsvorrichtung 1 nach Figur 4 kann so ausgelegt werden, dass neben einer Längsbewegung auch eine Drehbewegung der beiden Komponenten 2 und 3 zueinander zugelassen und durch ein Magnetfeld beeinflusst werden kann.

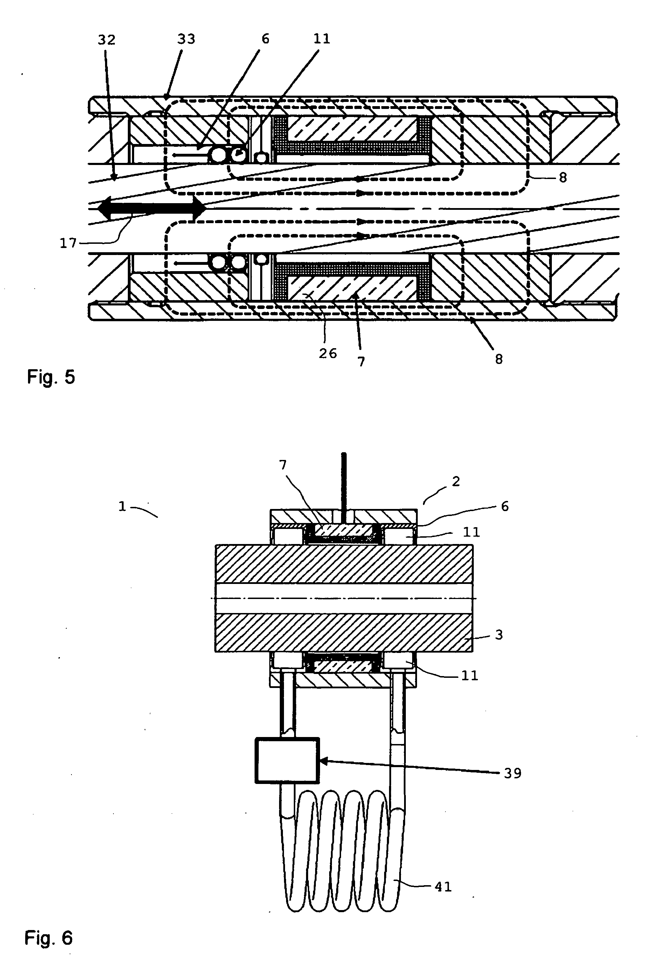

Figur 5 zeigt einen vergrößerten Querschnitt des Zentralbereiches der magnetorheologischen Übertragungsvorrichtung 1 gemäß Figur 4. Klar erkennbar ist die elektrische Spule 26 als Magnetfelderzeugungseinrichtung 7, um ein gezieltes Magnetfeld 8 zu erzeugen.

Radial zwischen der Stange 32 und der Komponente 3 sind Kugeln als Drehkörper 11 angeordnet, die in axialer Richtung gegenüber der Stange 32 bzw. der Komponente 3 beweglich vorgesehen sind und sich bei einer Relativbewegung der Komponente 2 und 3 zueinander bewegen und insbesondere in Rotationsbewegung versetzt werden können. Bei einer solchen Rotationsbewegung entstehen die spitzwinkligen Bereiche 10, die bei Aktivierung des Magnetfeldes 8 zu einer Verkettung der Partikel 19 des magnetorheologischen Fluids 20 als Medium 6 führen und somit die Relativbewegung der Stange 32 zur Komponente 3 verzögern oder erschweren oder sogar blockieren. Eine solche magnetorheologische Übertragungsvorrichtung 1 kann auch als Schwingungs- oder Stoßdämpfer oder dergleichen eingesetzt werden.

Figur 6 zeigt eine magnetorheologische Übertragungsvorrichtung 1 mit einer stehenden Komponente 2 und einer als Welle ausgeführten drehbaren Komponente 3, bei der zylindrische Drehkörper als Drehkörper 11 zwischen der Komponente 2 und der Komponente 3 angeordnet sind und von einem Medium 6 umgeben sind, welches auf ein Magnetfeld 8 einer Magnetfelderzeugungseinrichtung 7 reagiert.

Wird die magnetorheologische Übertragungsvorrichtung 1 in Figur 6 beispielsweise als Bremse eingesetzt, so wird die Bremsenergie in dem Medium 6 dissipiert. Häufiges und/oder starkes Bremsen kann dem Medium viel Energie zuführen, was zu einer erheblichen Aufheizung des Mediums bzw. Fluids 6 und der Drehkörper 11 führen kann. Um die entstehende Wärmeenergie abzuführen, kann eine Kühleinrichtung 41 vorgesehen sein, die beispielsweise über eine Pumpe 39 zwangsaktiviert werden kann. Die Pumpe 39 kann auch als eigenes Bauteil, welches die Relativbewegung nützt, in das Lager integriert werden. Vorteilhafter Weise wird zumindest ein Teil des Drehkörpers und/oder der Komponenten so gestaltet, dass eine Relativbewegung zumindest einen Teil des Mediums im Kühlkreislauf bewegt.

Ein weiterer vorteilhafter Effekt einer zwangsaktivierten Kühleinrichtung kann ein stetiges Durchmischen der Flüssigkeit und die zur Verfügungstellung von genügend MRF sein, wobei die Kühleinrichtung als Vorratsbehälter für die MRF-Flüssigkeit dienen kann.

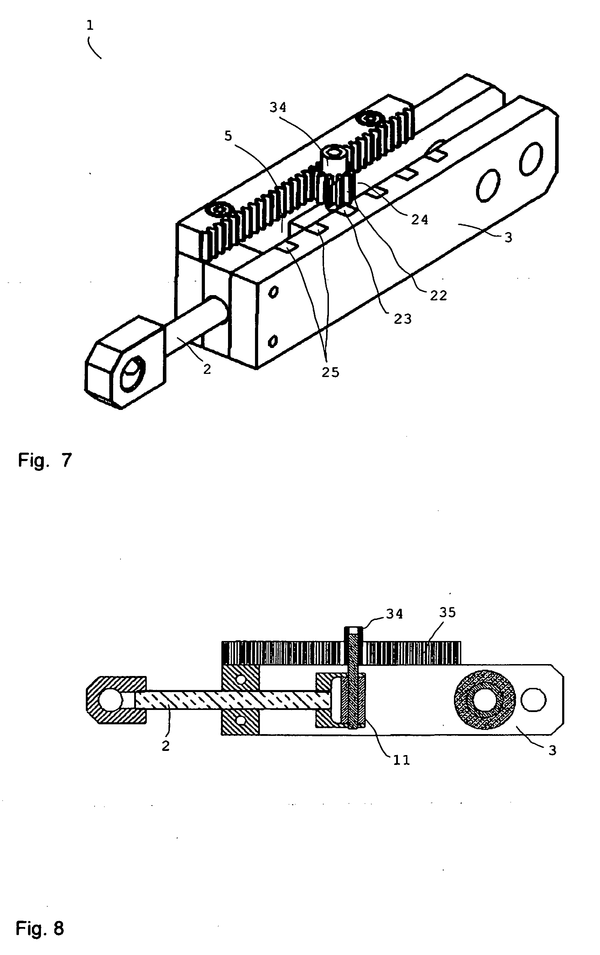

Figur 8 zeigt einen Querschnitt der magnetorheologischen Übertragungsvorrichtung 1 aus Figur 7. Die Komponente 2 weist einen drehbar aufgenommenen Drehkörper 11 auf, der mit einem Zahnrad 34 versehen ist. Das Zahnrad 34 kämmt mit einer Zahnstange 35 der Komponente 3. Wird die Komponente 2 relativ zur Komponente 3 bewegt, führt das zu einer Drehbewegung des Drehkörpers 11, da das Zahnrad 34 des Drehkörpers 11 mit der Zahnstange 34 der Komponente 3 kämmt. Ist der Drehkörper 11 von einem Medium 6 umgeben, welches durch ein Magnetfeld 8 beeinflussbar ist, kann durch Anlage beispielsweise eines externen Magnetfeldes ein magnetorheologisches Fluid 20 auf das Magnetfeld reagieren. Dadurch bildet sich zwischen den Platten der Komponente 3 und dem Drehkörper 11 jeweils ein spitzwinkliger Bereich 10 mit einer Keilform 16 aus, die eine weitere Relativbewegung der Komponenten 2 und 3 zueinander erschwert.

Das Zahnrad 34 und die Zahnstange 35 können je nach Anwendungsfall so dimensioniert werden, dass die Drehgeschwindigkeit der Relativgeschwindigkeit der Komponenten 2 und 3 zueinander entspricht bzw. erhöht oder gesenkt wird bzw. stark erhöht oder stark gesenkt wird.

Die Komponente 3 kann auch nur aus einer Platte bestehen, es ergibt sich dann nur ein spitzwinkliger Bereich 10 mit einer Keilform 16.

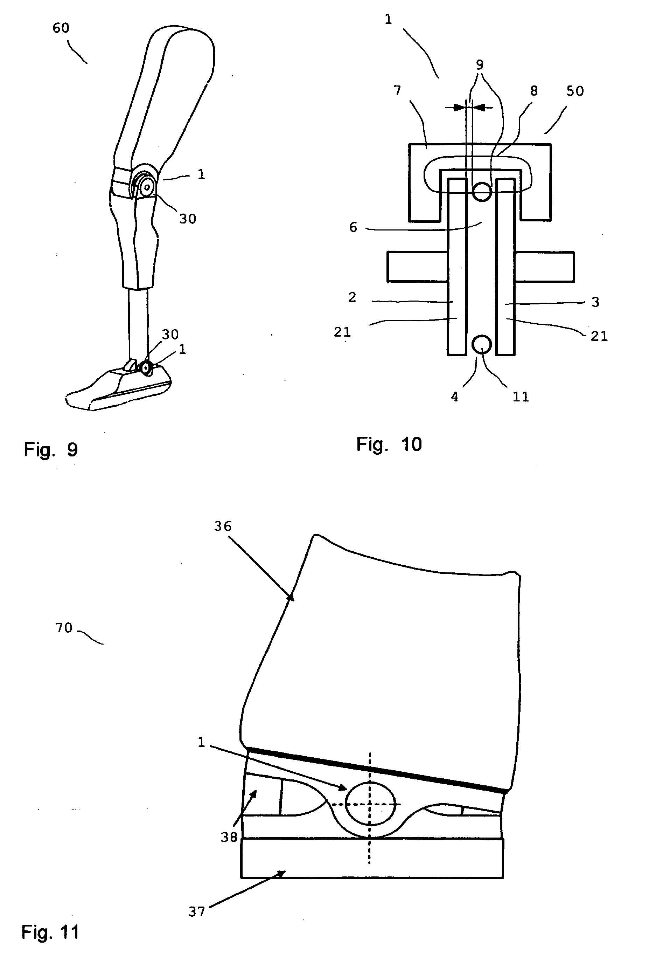

Figur 9 zeigt eine Prothese 60, bei der in das Knie- und das Fußgelenk jeweils magnetorheologische Übertragungsvorrichtungen 1 eingesetzt werden. Durch Aktivierung der entsprechenden Magnetfelder 8 kann eine Drehbewegung gedämpft oder blockiert werden, wodurch das Verharren in einer Position erleichtert und ein natürlicherer Bewegungsablauf ermöglicht wird.

Figur 10 zeigt in einer stark schematischen Darstellung einen Querschnitt durch eine Kupplung 50. Dabei sind zwei Kupplungsscheiben 21 vorgesehen, die in einem geringen Abstand zueinander angeordnet sind. Zwischen den Kupplungsscheiben ist eine Medium 6 vorgesehen, welches beispielsweise als magnetorheologisches Fluid 20 ausgeführt ist. Weiterhin sind zwischen den Kupplungsscheiben 21 Drehkörper 11 vorgesehen, die hier als Drehkörper 11 in Form von Kugeln 14 ausgebildet sind. Bei einer Relativbewegung der Kupplungsscheiben 21 zueinander werden die Drehkörper 11 in Drehbewegung versetzt. Bei Aktivierung eines Magnetfeldes 8 durch eine Magnetfelderzeugungseinrichtung 7 führt dies zur Bildung aktiver spitzwinkliger Bereiche 10, die eine weitere Relativbewegung der Kupplungsscheiben 21 zueinander erheblich hemmen.

Figur 11 zeigt ein weiteres Ausführungsbeispiel einer erfindungsgemäßen magnetorheologischen Übertragungsvorrichtung 1, die hier als Schuh 70 ausgebildet ist und nur schematisch dargestellt ist. Der Schuh 70 weist ein Oberteil 36 und eine Sohle 37 auf, die über ein Drehgelenk mit einer magnetorheologischen Übertragungsvorrichtung 1 miteinander verbunden sind. Um eine Grundposition vorzugeben, ist eine Federeinrichtung oder ein Schaumstoff 38 vorgesehen, der den Schuh 70 in seine Grundposition vorbelastet. Durch einen solchen Schuh kann eine Überpronation oder eine Supination flexibel ausgeglichen werden, in dem bestimmte Neigungswinkel zugelassen bzw. verhindert werden. Zur verbesserten Übersichtlichkeit der Darstellung werden Elektronik, Sensoren, Stromspeichereinheit usw. hier nicht dargestellt. Diese können vorzugsweise in die Sohle 37 integriert sein.

Da die Kraft auf die beiden Komponenten im Millisekundenbereich so angepasst werden kann, dass das Oberteil 36 gegenüber der Sohle 37 eine beliebige Neigung einnimmt, kann ein solcher Schuh 70 zum kontinuierlichen Ausgleich von Fehlstellungen des menschlichen Fußes eingesetzt werden. So kann sich durch eine Schrägstellung der Laufschuhsohle eine größere Unterstützung für den Innenfußbereich ergeben, was z.B. bei einer Überpronation vorteilhaft ist. Je nach Laufgeschwindigkeit, Untergrund und Muskelzustand, der auch maßgeblich durch Ermüdung beeinflusst wird, passt sich die Fußraumform an die neuen Gegebenheiten an, sodass der Läufer mit einem erfindungsgemäßen Schuh 70 eine gute Position im Laufschuh einnimmt. Denkbar ist es auch, dass bei größeren geforderten Verstellwegen der Verstellvorgang auf mehrere Schritte aufgeteilt wird. Gegebenenfalls kann auch ein Dämpfungsmaterial in den Schuh integriert werden. Sensoren können den Istzustand erfassen und mittels einer Steuer- und/oder Regelelektronik Anpassungen vornehmen. Es ist auch möglich, einen Aktor an das Ende des Laufschuhs anzuordnen und nicht nur unter den Laufschuh bzw. unter die Ferse. Praktisch kann dann ein Einstellknopf oder Drehknopf am Ende des Laufschuhs angeordnet sein.

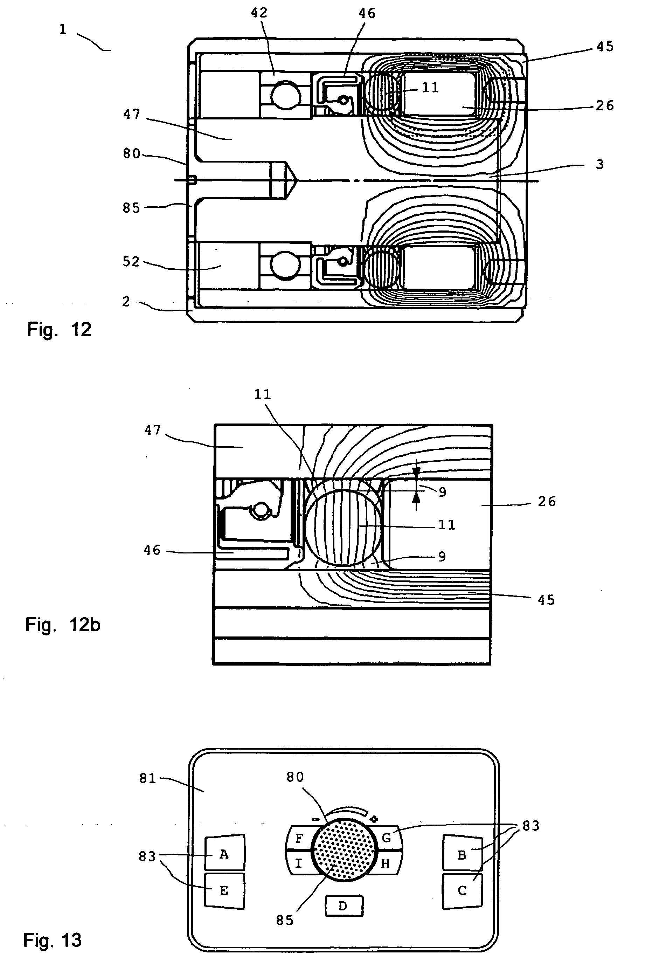

Fig. 12 zeigt einen Bedienknopf oder Drehknopf 80 mit einer erfindungsgemäßen magnetorheologischen Übertragungsvorrichtung 1 in einem schematischen Querschnitt. Das Gehäuse 45 als Komponente 2 kann z.B. an einem Gerät fest angebracht sein. Die Welle 47 als Komponente 3 ist mit dem Drehteil 85 verbunden. Beide Komponenten 2 und 3 sind über Lager 42 gegeneinander drehbar gelagert. Ein dünner Spalt als freier Abstand 9 befindet sich zwischen dem Drehkörper 11 und dem Gehäuse 45 und auch zwischen dem Drehkörper 11 und der Welle 47. Der die Drehkörper 11 umgebende Raum und gegebenenfalls nahezu der gesamte Innenraum kann mit einem magnetorheologischen Fluid als Medium 6 gefüllt sein. Ein Dichtring 46 wirkt als Dichtung gegenüber dem Lager 42, welches so vor den Partikeln in dem magnetorheologischen Fluid geschützt ist.

Bei Aktivierung der Spule 26 wird ein Magnetfeld 8 erzeugt, welches, wie die beispielhaft eingezeichneten Feldlinien zeigen, durch die Drehkörper 11 durchtritt und hier im Wesentlichen sonst innerhalb des Gehäuses 45 und der Welle 47 verläuft. Bei aktiviertem Magnetfeld der Spule 26 wird ein entsprechender Widerstand in dem Medium 6 bzw. dem MR-Fluid erzeugt, sodass beim Drehen des Drehteils 85 ein entsprechender Widerstand spürbar ist. Möglich ist auch ein z.B. zeitlich gepulster oder pulsierender Betrieb, durch den beim Drehen ein pulsierender Widerstand und somit eine Rasterung spürbar wird.

Die jeweils aktuelle Winkelposition kann über einen Drehgeber 52 erfasst werden. Dadurch können abhängig von der Ansteuerung je nach Position, Drehwinkel, Winkelgeschwindigkeit etc. beliebige haptische Signale ausgegeben werden. Der Drehgeber 52 kann auch um einen Drehmomentsensor ergänzt werden.

Fig. 12b zeigt einen schematischen vergrößerten Ausschnitt aus Fig. 12, in welchem ein Drehkörper 11 vollständig und ein dahinter angeordneter Drehkörper 11 nur teilweise zu sehen ist, da er teilweise durch den vorderen Drehkörper 11 und ein wenig durch die Welle 47 verdeckt wird. Der Spalt 9 bzw. freie Abstand oberhalb und unterhalb des Drehkörpers 11 ist klar erkennbar. Die freien Abstände können radial nach innen und radial nach außen gleich sein, müssen es aber nicht. Der freie Abstand 9 entspricht in der Lagerterminologie dem Laufspiel. Das doppelte Laufspiel entspricht bei einem Lager der Lagerluft.

Fig. 12b ist nochmals zu entnehmen, dass auf der Welle 47 und auch in dem Gehäuse 45 als Komponenten 2, 3 keine separaten Laufflächen vorgesehen sind. Die Übertragung eines Kupplungs- oder Bremsmomentes erfolgt über die Drehkörper und die verstärkende Keilwirkung des MRF.

Fig. 13 zeigt einen Drehknopf 80, der an einem Display bzw. an einer Anzeige 81 angeordnet ist. An der Anzeige 81 können auch weitere Bedienelemente oder Tasten 83 vorgesehen sein. Die Anzeige 81 kann auch als Touchscreen berührungsempfindlich ausgeführt sein und z.B. Knöpfe oder Tasten 83 darstellen. Möglich ist es z.B., dass die Tasten 83 programm- und/oder auswahlabhängig dargestellt oder aktiviert werden, um dem Benutzer die individuelle Bedienung zu erleichtern.

Zweidimensionale Haptikknöpfe oder Drehknöpfe 80 können auch mit einem zusätzlichen MRF Schermodus hergestellt werden.

Ein MRF Haptikknopf kann sehr kleinbauend für Stelleinrichtungen in Spiegelreflexkameras und sonstigen Fotoapparaten, sowie Spielekonsolen und sonstiger handgehaltenen Computern ausgeführt werden. Solche kleinbauende MRF Kopplungseinrichtungen sind wegen des geringen Platzbedarfs und des geringen Stromverbrauchs im Bereich von Milliwatt oder darunter für Kameras und andere Outdooranwendungen gut geeignet. Das Raster ist situationsabhängig einstellbar.

Dreidimensionale Bewegungselemente mit variabler Haptik und robuster und präziser Lagerung sind grundsätzlich schwierig herzustellen und somit nicht preisgünstig. Die Kombination z.B. einer pendelfähigen Anordnung der Drehkörper mit einer magnetorheologischen Flüssigkeit ist hingegen sehr kostengünstig herstellbar.

Auch ein vierdimensionaler Drehknopf, der z.B. in drei Richtungen verschoben und zusätzlich noch gedreht werden kann, kann zur Verfügung gestellt werden.

Die Kombination eines 3D-Knopfes mit einer Längsverstellung eines MRF-Keils ergibt so ein 4D Betätigungselement. Mit einer Felderzeugungseinheit können alle vier Bewegungsrichtungen beeinflusst bzw. variiert werden.

Der Einsatz solcher Haptikknöpfe ist auch an berührungsempfindlichen Anzeigen wie Touchdisplays in Mobiltelefonen, PDA's, Smartphones, tragbaren und stationären Computern und Bildschirmen, Spielkonsolen, TabletPC's, Laptops usw. möglich. Dazu wird dort wenigstens ein haptisches Element in Form z.B. eines Drehknopfes vorgesehen.

Ein solches Haptikelement 1 kann auch klapp-/schwenk- bzw. verschiebbar ausgeführt werden und z.B. aus einer Ruheposition am Rand in eine Position über dem Display verschoben werden. Sobald das Haptikelement über dem Display ist, kann sich die Anzeige im Display ändern, d.h. ein Menü erscheint unter oder um den Knopf herum.

Statt einem kinematischen und z.B. parallelogrammartigen Schwenkmechanismus kann auch ein elastisches/verformbares Element verwendet werden, welches z.B. als biegsamer und halbsteifer Arm aus gewendeltem Metallschlauch in Form eines Schwanenhalses bestehen kann. Ein Vorteil ist, dass der Benutzer nicht immer in den Bildschirm greifen muss, was die Verschmutzung reduziert. Außerdem geht die Verstellung und z.B. das Zoomen schneller: Mit einem Finger in den Bildschirm greifen und mit anderen Finger den Drehregler bewegen kann z.B. einen Zoomvorgang auslösen. Das gleiche gilt für die Lautstärke, das Groß-Kleinschreiben oder die Auswahl von Sondertasten oder einer zweiten Ebene beim Tippen.

So kann der Benutzer auch auf eine eigene Bedienleiste mit dem einen Finger drücken, um die Art der gewünschten Betätigung auszusuchen. Mit dem Drehregler führt er dann die gewünschte Aktion durch. Der Raster des Drehregler passt sich dann automatisch an, so z.B. "Ein"-"Aus" oder eine Lautstärkeregelung mit einem Raster evtl. mit einem dynamischem Anschlag. Wenn der Bildschirm während der Betätigung (Touchdisplay) gedreht wird (z.B. wie bei Mobiltelefonen oder handegehaltenen Computern - 90° von Hochformat auf Längsformat), dann passt sich der Raster automatisch an, d.h. er dreht sich mit. Wäre z.B. beim Hochkanthalten der Einstellbereich von 6:00 Uhr bis 12:00 Uhr, dann wäre dieser ohne Anpassung beim Drehen um 90° im Uhrzeigersinn von 12:00 Uhr bis 6:00 Uhr. Dies gilt auch, wenn das Display im Knopf selber eingebaut ist. Ein solches haptisches Element kann in alle oder einzelne Richtungen haptisch sein (nur drehen, drehen und drücken; Joystick etc.). Je nach gewählter Aktion verstellt sich die Haptik.

Ein Vorteil kann sich auch beim Auswählen von z.B. einer Liste wie einer Telefonbuchliste ergeben, da solche Einträge für große Finger oft zu klein zum "Zielen" sind.

Vorteile ergeben sich auch im Dunkeln oder für Leute mit Brille, welche diese gerade nicht tragen. Über den haptischen Drehregler erhält man eine Rückkopplung und der Benutzer weiß, was er macht, wenn es z.B. gerade dunkel ist.

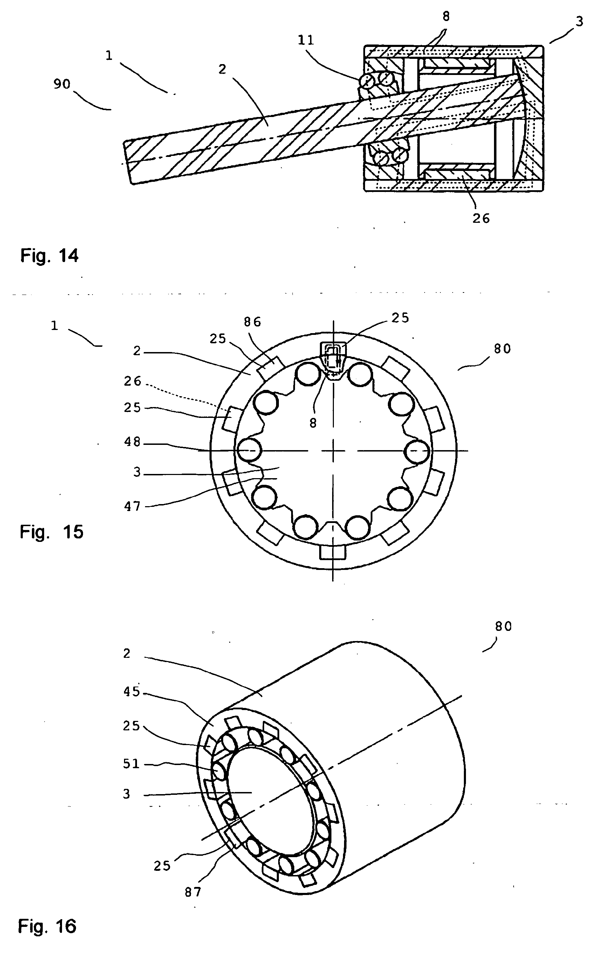

Fig. 14 zeigt einen solchen 3D-Knopf als Joystick 90, der in verschiedene Richtungen schwenkbar vorgesehen ist. Durch sensor- oder zeitgesteuerte Aktivierung der Spule 26 kann ein haptisches Raster realisiert werden. Beispielhaft sind Magnetfeldlinien 8 eingezeichnet. Hierbei treten sehr geringe Kräfte auf, wodurch die MRF Partikel nicht durch hohe Flächenpressungen beschädigt werden.

In Fig. 15 ist eine schematische Ansicht eines Drehknopfes 80 als magnetorheologische Übertragungsvorrichtung 1 dargestellt, welcher eine äußere Komponente 2 und eine innere Komponente 3 aufweist. In einem Spalt 86 zwischen den beiden Komponenten 2 und 3 befindet sich ein MRF. Von der als Welle 47 ausgeführten Komponente 3 stehen Erhebungen 49 ab, die als radiale Vorsprünge wirken. Zusätzlich sind in vorbestimmten Winkelabständen Dauermagnete 25 als Magnetfelderzeugungseinrichtungen bzw. Magneteinheiten oder Vorsprünge an der Komponente 2 vorgesehen. Die Magnetfelder der Dauermagneten 25 führen zu einer lokalen Haufenbildung in dem Medium 6. Dadurch wird die Wirkung noch verstärkt, sodass erhebliche Drehmomente abgefangen werden können. Die Anordnung der Magneteinheiten 87 führt zu einer fühlbaren Rasterung beim Drehen des Drehknopfes 80. In Fig. 15 werden die Erhebungen teilweise durch separate Drehkörper 11 gebildet, die in entsprechenden Ausnehmungen 88 der Erhebungen 49 angeordnet sind, und sich vorzugsweise darin drehen können.

Fig. 16 zeigt einen Drehknopf 80, bei dem zwischen der inneren Komponente 3 und der äußeren Komponente 2 Drehkörper 11 bzw. Rollkörper 51 vorgesehen sind.

Es ist möglich, dass das Magnetfeld radial durch den Spalt und/oder die Drehkörper 51 geht. Es ist auch möglich, dass das Magnetfeld axial durch den Spalt und/oder die Drehkörper 51 geht, so z.B. axial auf einer Seite hinein und auf der anderen Seite wieder hinaus. Weiterhin ist auch eine Kombination der vorgenannten Alternativen möglich.

In konkreten Ausführungen kann der Käfig kann auch mit einer reibungserhöhenden Schicht versehen sein oder aus einem speziellen Material gefertigt sein, wodurch sich der Momentenunterschied zwischen "Eingeschaltet" und "Ausgeschaltet" erhöht. Statt rotativ kann auch eine lineare Betriebsweise möglich sein. Es können auch Kugelumlaufbüchsen oder Linearlager mit Käfig bzw. Linearkugellager eingesetzt werden, um eine tragkräftige Abstützung zu erzielen.

Der gesamte Aufbau ist sehr einfach und kostengünstig herstellbar, da solche Drehkörper Massenartikel sind.

In allen Fällen können Wirbelstromeffekte bei schnell drehenden Drehkörpern berücksichtigt werden.

Es ist möglich, eine oder beide Komponenten wenigstens teilweise und/oder einzelne oder alle die Drehkörper 11 bzw. 51 aus einem magnetorheologischen MR-Kunststoff oder magnetorheologischen Elastomer herzustellen, der je nach Feldstärke seine Form ändert und so die Drehkörper klemmt. Ein solches System kann komplett ohne Dichtung auskommen.

Eine Ausführungsform weist in Kunststoff gebundenen MRF wie Schaumstoff oder einen Schwamm auf. Als MR-Pulver könnte ein fester Schmierstoff, so z.B. Lithium, Graphit oder MoS2 oder dergleichen mit Carbonyleisenpulver gemischt werden.

Zur Lagerung gegeneinander drehbarer Komponenten 2, 3 können konventionelle Wälzlager oder Gleitlager eingesetzt werden. In bestimmten Fällen, z.B. bei sehr geringen Lasten, kann auch auf separate Lager verzichtet werden.

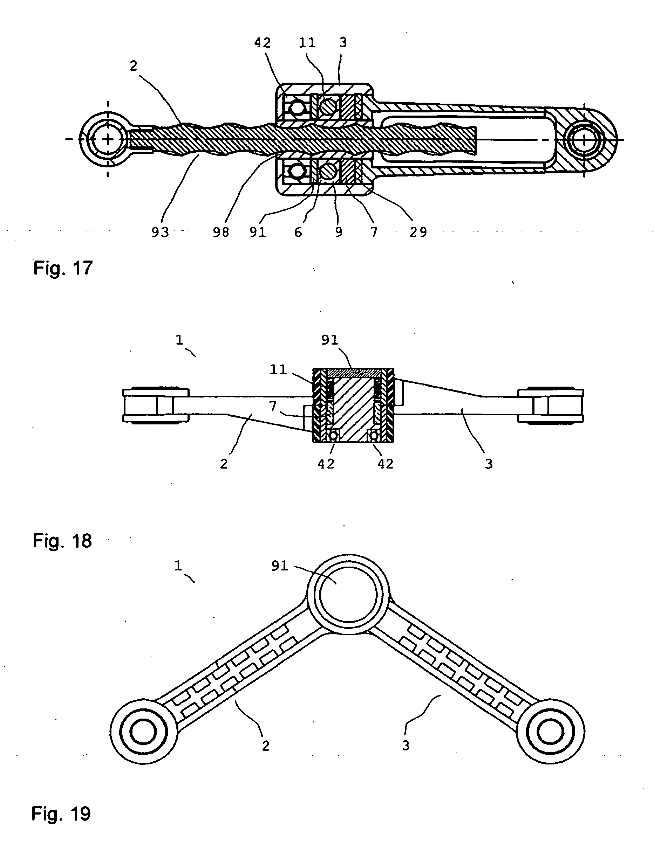

Fig. 17 zeigt eine Ausführungsform einer magnetorheologischen Übertragungsvorrichtung 1, bei der eine Linearbewegung wie ein Hub oder dergleichen über eine Spindel wie z.B. eine Kugelspindel oder eine einfache Spindel in eine Drehbewegung umgewandelt wird. Die eine Komponente 2 ist als Gewindespindel 93 ausgeführt und wird linear bewegt. Darauf sitzt eine Spindelmutter 98. Eine Linearbewegung wird in eine Drehbewegung umgewandelt. Es können Lager 42 zur Lagerung vorgesehen sein, die dann über eine Dichtung 91 abgedichtet sind.

Die Drehkörper 11 sind in einem Spalt 5 angeordnet, der mit MRF gefüllt ist. Der Spalt kann einem Magnetfeld der Magnetfelderzeugungseinrichtung 7 ausgesetzt werden, wodurch die Relativbewegung der Komponenten 2 und 3 gedämpft und wiederum die Hubbewegung beeinflusst wird. Möglich ist der Einsatz in unterschiedlichen Anwendungen, beispielsweise in Sportgeräten oder Waschmaschinen als Dämpfer.

Optional kann die magnetorheologische Übertragungsvorrichtung 1 als MRF Bremse auch um einen Drehgeber 29 erweitert werden. Das Erfassen einer Drehbewegung ist kostengünstiger und einfacher umzusetzen wie das Erfassen einer Längsbewegung. Dies gilt auch für das Abdichten. Zusätzlich oder statt dem Drehgeber 29 kann auch ein Drehmomentsensor eingesetzt werden.

In allen Ausgestaltungen kann ein bzw. wenigstens ein Permanentmagnet vorgesehen sein, der motorisch oder von Hand verstellbar ist. Möglich ist auch der Einsatz einer verschiebbaren Abschirmung. In allen Fällen ist ein mechanisches Einstellen der Bremswirkung und somit des Keileffekts möglich. Das kann z.B. zur Kompensation von physikalischen Größen wie Temperatur, Druck, Drehzahl oder dergleichen dienen. Die Betätigung kann direkt oder über z.B. einen Bowdenzug erfolgen. Die Verstellung kann stufenlos sein.

Fig. 18 und 19 zeigen eine MRF Übertragungsvorrichtung 1 in einem Kniehebel in zwei unterschiedlichen Ansichten.

Der Kniehebel verfügt über zwei Arme, die hier die drehbar zueinander angeordneten Komponenten 2 und 3 bilden. Lager 42 können zur tragfähigen Abstützung vorgesehen sein. Eine Magnetfelderzeugungseinrichtung 7 dient zur Erzeugung eines Magnetfeldes in dem Spalt, in dem ein MRF und Drehkörper 11 vorhanden sind. Durch den genügend großen aber nicht zu großen freien Abstand 9 kann wahlweise ein sehr hohes Brems- bzw. Kupplungsmoment aufgebaut werden. Eine Dichtung 91 dichtet nach außen hin ab. Die MRF Übertragungsvorrichtung 1 kann auch um einen Drehgeber und/oder einen Drehmomentsensor und/oder andere Sensoren ergänzt werden.

Solche magnetorheologischen Übertragungsvorrichtungen können für Beschläge bei Möbeln eingesetzt werden. Beispielsweise als Lineareinheit für Schubladenführungen usw. Durch die Drehkörper erfolgt dabei eine hinreichende Führung, während gleichzeitig die Auszugskraft variierbar ist.

Generell können magnetorheologische Übertragungsvorrichtungen 1 mit Keileffekt als variable und einstellbare Bremse bei Küchen und anderen Möbeln eingesetzt werden. Das Schwenken wie z.B. das Öffnen von Türen oder Klappen bei Möbeln kann auf bestimmte Bereiche eingeschränkt werden, während ein schnelles Öffnen möglich ist.

Die Einstellung kann z.B. über verdrehbare Permanentmagneten oder elektrisch erfolgen oder aber über einen Hebel oder Drehhebel.

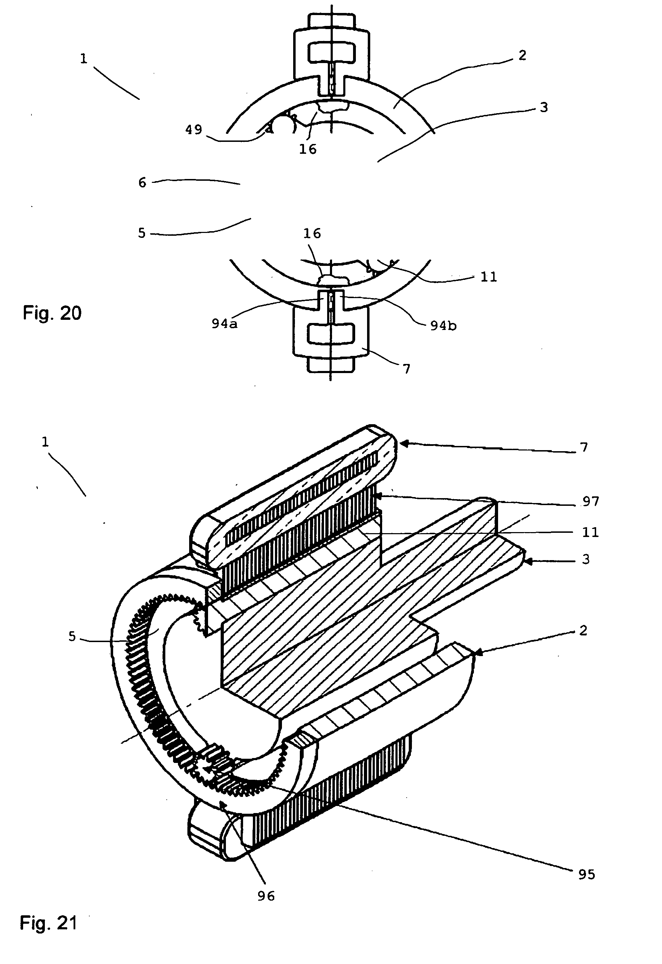

Fig. 20 zeigt eine MRF Übertragungsvorrichtung 1 in einer stark schematischen Vorderansicht und Fig. 21 zeigt die Übertragungsvorrichtung 1 aus Fig. 20 in einem stark schematischen Teilquerschnitt.

Eine hier innere Welle 3 ist drehbar in einer äußeren Hohlwelle als Komponente 2 angeordnet. Die äußere Hohlwelle 2 kann feststehend ausgeführt sein. An der Hülse 2 sind hier zwei Elektromagnete mit jeweils einem Blechpaket 97 als Magnetfelderzeugungseinrichtungen 7 angeordnet. Es können auch drei oder mehr Elektromagnete vorgesehen sein. Möglich ist es auch, dass nur ein Magnet vorgesehen ist. Anstelle von Elektromagneten können zumindest teilweise Permanentmagnete eingesetzt werden.

Zwischen den Komponenten 2 und 3 ist ein Spalt 5 vorgesehen, der mit einem MRF gefüllt ist. In dem Spalt als Kanal 5 sind hier zwei Drehkörper 11 angeordnet, die hier als im Wesentlichen zylindrische Drehkörper ausgebildet sind. An einem Ende können die zylindrischen Drehkörper 11 mit Ritzeln 95 versehen sein, die in einer Verzahnung 96 zwangsgeführt werden, sodass für eine kontinuierliche Drehbewegung der Drehkörper 11 gesorgt wird. Dabei kann der Bereich der Verzahnung 96 im Wesentlichen feld- und MRF-frei sein. Die Kopplung erfolgt hier vorwiegend über den sich im Spalt 5 bildenden Keil. Die Übertragungsvorrichtung kann mit verschiedenen Verzahnungen bzw. Übersetzungen, aber auch ohne, zur Zwangsdrehung der Drehkörper 11 ausgeführt werden.

Die in Fig. 20 abgebildete Magnetfelderzeugungseinrichtung 7 weisen jeweils einen Nordpol 94a und einen Südpol 94b auf, die mit geringem Abstand voneinander an den Kanal 5 mit dem darin befindlichen MRF grenzen. Die Magnetfeldlinien verlaufen durch die beiden Pole 94a und 94b in den Ringspalt als Kanal 5 hinein und bilden dort Haufen aus sich verfestigendem MRF, wenn das Feld angelegt ist. Die im Bereich des Ringspaltes zylindrisch ausgebildeten Drehkörper laufen bei der erzwungenen Drehbewegung auf die MRF-Strukturen auf. Der dann entstehende Keileffekt und der spitzwinklige Bereich an den Drehkörpern führt zu einem sehr starken Bremsmoment.

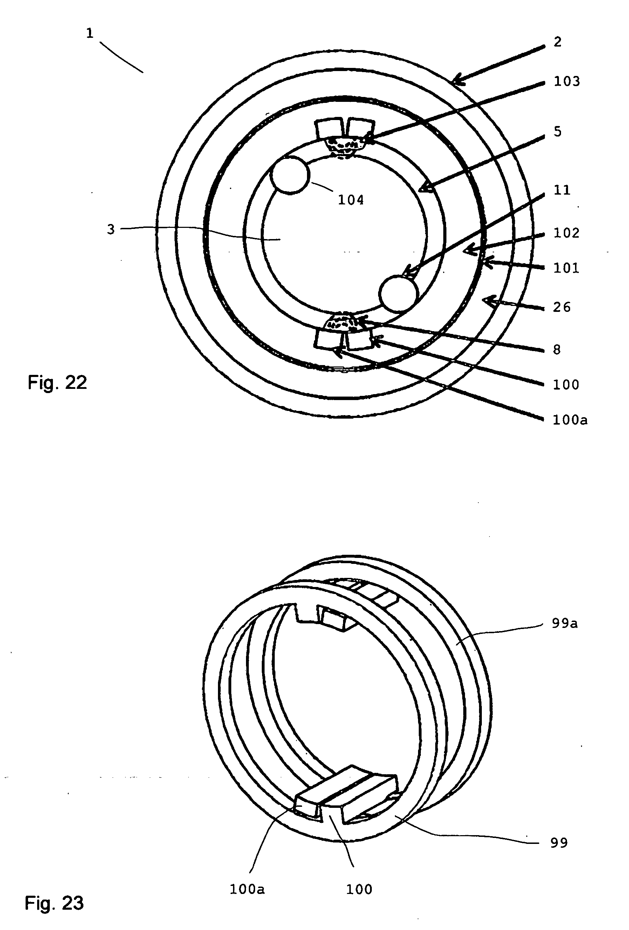

Eine solche magnetorheologische Übertragungsvorrichtung 1 als Keilkupplung nach Fig. 20 kann in einer Abwandlung nach Fig. 22 auch eine in einem nicht drehenden Gehäuse befestigte Spule aufweisen, die wahlweise ein Magnetfeld erzeugt. Das Magnetfeld wird über Polscheiben 99 und 99a geleitet und schließt sich über Finger 100 und 100a, die axial an den Polscheiben 99 und 99a befestigt sind. Zwischen den Fingern 100 und 100a der Polscheiben 99 und 99a wird im MRF-Kanal 5 bzw. Spalt das MRF zu Haufen 103 verfestigt. Die Polscheiben 99 und 99a rotieren mit der Antriebswelle 2. Treffen die verfestigten MRF-Bereiche 103 auf die in den halbmondförmigen Ausnehmungen 104 angeordneten Drehkörper 11 auf, die an der Abtriebswelle 3 befestigt sind, werden diese abhängig vom Magnetfeld kurz mitgerissen.

Durch die Übertragung des Feldes 8 radial von außen über den Luftspalt 101 in die Polscheiben 99 und 99a können die Spulen feststehend vorgesehen sein. Eine Übertragung der Stromversorgung über Schleifringe ist nicht nötig. Der magnetische Fluss wird über die Polscheiben 99 und 99a und deren Polfinger 100 und 100a in den Kanal 5 eingebracht, wo sich die Feldlinien schließen und zur Bildung der Haufen 103 führen, die keilförmig mit den Drehkörpern 11 zusammenwirken.

Die Polscheiben 99 und 99a werden in einem hier aus Kunststoff bestehenden Ring gehalten, der über einen Luftspalt 101 von der außen drehenden Komponente 2 bzw. Welle getrennt ist.

Bevorzugt sind alle Komponenten ferromagnetisch, außer dem insbesondere als Kunststoffteil ausgeführtem Teil zwischen den Polscheiben 99 und 99a und der An- und Abtriebswelle 2 und 3.

Die zwei "Fingerpaare" (MRF Haufen) können gegebenenfalls einzeln und unabhängig voneinander angesteuert werden.

In allen Fällen ist es auch möglich, magnetorheologische Übertragungsvorrichtungen 1 mit Keileffekt in eine Radnabe eine Fahrzeuges und z.B. eines Fahrrades einzubauen, um z.B. zu Bremsen. Die benötigte elektrische Energie kann Strom direkt von eingebautem Dynamo beziehen, der insbesondere parallel dazu geschaltet ist. Über den Dynamo kann eine Energierückgewinnung erfolgen. Bei einer (Voll-) Bremsung kann die magnetorheologische Übertragungsvorrichtung 1 als MRF Bremse dienen. Das System wird aufeinander abgestimmt. Da eine solche MRF Bremse rein elektrisch funktioniert und schnell reagiert, ist der Einsatz gut möglich. Entsprechende Laufflächen für die Drehkörper sind vorhanden. Ein Großteil könnte so über einen Radnabendynamo gebremst werden. Zur Betätigung am Lenker wird nur ein Stromkabel in Verbindung mit z.B. einem Potentiometer benötigt oder die Übertragung erfolgt drahtlos.

Möglich ist der Einsatz auch als Bremse bei Fitnessgeräten oder als Kupplung oder Bremse an Rudermaschinen. Das Prinzip kann auch als Keilkupplung zum Einkuppeln von Aggregaten und insbesondere Nebenaggregaten bei Kraftfahrzeugen genommen werden. Evtl. können zwei MRF-Haufen relativ dicht beieinander vorgesehen werden, damit das System bei einem Drehrichtungswechsel nicht gleich öffnet.

Bei Überlast öffnet eine solche Kupplung automatisch. Es werden gegebenenfalls keine Schleifringe zur Stromübertragung benötigt. Das kann z.B. über Remanenz erfolgen.

Wenn bei einer solchen MRF-Keilkupplung der Drehkörper 11 beim ersten Einkupplungsversuch noch über den MRF Keil bzw. MRF Haufen springt, so wird das zuvor stehende Teil beschleunigt und beim zweiten Versuch wird der Einkupplungsvorgang erleichtert. Möglich ist auch der Einsatz als Freilauf, indem ein schnelles Erkennen der Drehrichtung erfolgt und indem das Feld ausgeschaltet wird, wenn eine andere Drehrichtung erkannt wird.

Möglich ist auch der Einsatz bei einer Kupplung in Fräsmaschinen, wobei z.B. ein Auskuppeln erfolgt, wenn während des Laufens der Maschine der Notausschalter gedrückt wird. Möglich ist es auch, die Kupplung bei Überlast schlagartig zu trennen. Normale (MRF-) Kupplungen reduzieren das Moment nicht schlagartig.

In allen Fällen und Ausgestaltungen kann der Keil und/oder das Magnetfeld auch am Innenring erzeugt werden und nicht nur am Außenring.

Die Drehkörper 11 und Käfige können ganz oder teilweise ferromagnetisch und paramagnetisch oder diamagnetisch ausgeführt sein. Möglich ist auch eine vollkuglige Ausführung und eine Ausführung, bei der alle Teile aus dem gleichen Werkstoff bestehen. Möglich ist es auch, dass einige Drehkörper ferromagnetisch sind und z.B. aus Stahl bestehen während andere aus Kunststoff bestehen. Möglich ist auch der Einsatz von Drehkörpern und Kugeln mit verschiedenen Durchmessern.

Vorzugsweise kann die erfindungsgemäße magnetorheologische Übertragungsvorrichtung 1 auch zu einer Drehzahlerkennung und insbesondere Drehzahlregelung eingesetzt werden.

Über eine Pulsweitenmodulation (PWM) kann das Moment drehzahlabhängig eingestellt werden. Große axiale und radiale Kräfte können über einen schrägen Spreizdorn erzeugt werden. Die Partikel können eine runde, stäbchenförmige oder jede andere Form haben.

Möglich ist es auch, magnetorheologische Elastomere einzusetzen. Beispielsweise kann wenigstens eine Fläche auch ein magnetorheologisches Elastomer sein. Grundsätzlich kann eine Komponente mit einem magnetorheologischen Elastomer versehen sein. Möglich ist auch eine Beschichtung wenigstens eines Drehkörpers 11 und/oder wenigstens einer der Komponenten 2, 3 mit einem magnetorheologischen Elastomer.

Die magnetorheologische Übertragungsvorrichtung 1 kann auch als Ventil ausgeführt werden, wobei ein Drehkörper 11 oder mehrere Drehkörper 11 den Kanal versperren.

Eine magnetorheologische Übertragungsvorrichtung kann auch für den Einsatz einer magnetorheologischen Flüssigkeit vorgesehen sein, die ein Produkt der Firma BASF ist, wie insbesondere das Produkt "Basonetic".

Die rheologische Flüssigkeit kann aus verschiedensten Bestandteilen bestehen, welche einzeln oder in Kombination sein können: Fe, Kohlenstoffstahl, NdFeB (Neodymium), Alnico, Samarium, Cobalt, Silizium, Kohlefaser, rostfreier Stahl, Polymere, Soda-lime glass, Kalknatronglas, Keramic und nicht magnetische Metalle und dergleichen mehr. Dimorphische magnetorheologische Flüssigkeiten mit Nanotubes oder/und Nanowires sind auch möglich.

Die Trägerflüssigkeit kann insbesondere aus den folgenden Bestandteilen oder einer Kombination daraus bestehen: Öle und vorzugsweise synthetische oder nicht synthetische Öle, Hydrauliköl, Glycol, Wasser, Fette und dergleichen mehr.

**Bezugszeichenliste:**

| | | | |
|---|---|---|---|
| 1 | Einrichtung | 39 | Pumpe |
| 2, 3 | Komponente | 40 | Bremse |
| 4 | separates Teil | 41 | Kühleinrichtung |
| 5 | Kanal | 42 | Lager |
| 6 | Medium | 45 | Gehäuse |
| 7 | Magnetfelderzeugungseinrichtung | 46 | Dichtring |
| | | 47 | Welle |
| 8 | Feld | 49 | Erhebung |
| 9 | freier Abstand | 50 | Kupplung |
| 10 | spitzwinkliger Bereich | 51 | Rollkörper |
| 11 | Drehkörper | 52 | Drehgeber |
| 12 | Drehachse | 60 | Prothese |
| 13 | Drehkörper | 70 | Schuh |
| 14 | Kugel | 80 | Bedienknopf |
| 15 | Zylinder | 81 | Anzeige |
| 16 | Keilform | 82 | Touchscreen |
| 17 | Richtung der Relativbewegung | 83 | Taste |
| | | 84 | Lautsprecher |
| 18 | Richtung der Relativbewegung | 85 | Drehteil |
| | | 86 | Spalt |
| 19 | magnetische Partikel | 87 | Magneteinheit |
| 20 | Fluid | 88 | Ausnehmung |
| 21 | Scheibe | 90 | Joystick |
| 22 | Außenseite | 91 | Dichtung |
| 23 | Vorsprung | 92 | Laufspiel |
| 24 | Verzahnung | 93 | Gewindespindel |
| 25 | Dauermagnet | 94a | Nordpol |
| 26 | Spule | 94b | Südpol |
| 27 | Steuereinrichtung | 95 | Ritzel |
| 28 | Energiespeicher | 96 | Verzahnung |
| 29 | Sensor | 97 | Blechpaket |
| 30 | Lager | 98 | Spindelmutter |
| 31 | feststehende Kom¬ponente | 99 | Polscheibe |
| 32 | Stange | 99a | Polscheibe |
| 33 | Außenrohr | 100 | Finger |
| 34 | Zahnrad | 100a | Finger |
| 35 | Zahnstange | 101 | Luftspalt |
| 36 | Oberteil Schuh | 102 | Kunststoffring |
| 37 | Sohle | 103 | Haufen |
| 38 | Schaumstoff | 104 | Aufnahme |

## Patentansprüche

1. Magnetorheologische Übertragungsvorrichtung (1) mit wenigstens zwei koppelbaren Komponenten (2, 3), deren Kopplungsintensität beeinflussbar ist, wobei zur Beeinflussung der Kopplungsintensität wenigstens ein Kanal vorgesehen ist, wobei der Kanal (5) ein durch ein Magnetfeld beeinflussbares magnetorheologisches Medium (6) mit magnetisch polarisierbaren Partikeln (19) enthält, und wobei wenigstens eine Magnetfelderzeugungseinrichtung (7) zur Erzeugung wenigstens eines Magnetfeldes (8) in dem Kanal (5) vorgesehen ist, um mit dem Magnetfeld (8) das magnetorheologische Medium (6) in dem Kanal (5) zu beeinflussen, und wobei
in dem Kanal (5) wenigstens ein Drehkörper (11) vorgesehen ist **dadurch gekennzeichnet, dass** ein freier Abstand (9) zwischen dem Drehkörper und wenigstens einer der Komponenten (2, 3) wenigstens zehnmal so groß ist wie ein typischer mittlerer Durchmesser der magnetisch polarisierbaren Partikel (19) in dem magnetorheologischen Medium (6), und dass zwischen dem Drehkörper (11) und wenigstens einer der Komponenten (2, 3) wenigstens ein spitzwinkliger und das magnetorheologische Medium (6) enthaltender Bereich (10) vorgesehen ist, der mit dem Magnetfeld (8) der Magnetfelderzeugungseinrichtung (7) beaufschlagbar ist, um die Partikel (19) wahlweise zu verketten und/oder mit dem Drehkörper (11) zu verkeilen oder freizugeben.

2. Magnetorheologische Übertragungsvorrichtung (1) nach Anspruch 1, wobei der spitzwinklige Bereich (10) zwischen dem Drehkörper (11) und einer der Komponenten (2, 3) sich in Richtung der Relativbewegung (17, 18) der Komponente (2,3) relativ zu dem Drehkörper (11) verjüngt.

3. Magnetorheologische Übertragungsvorrichtung (1) nach wenigstens einem der vorhergehenden Ansprüche, wobei der Drehkörper (11) als separates Teil (4) zwischen der ersten und der zweiten Komponente (2, 3) ausgebildet ist.

4. Magnetorheologische Übertragungsvorrichtung (1) nach wenigstens einem der vorhergehenden Ansprüche, wobei die eine Komponente (3) als äußere Komponente (3) die andere Komponente (2) als innere Komponente (2) umgibt und/oder wobei die beiden Komponenten (2, 3) über wenigstens ein separates Lager (42) gegenüber einander gelagert sind.

5. Magnetorheologische Übertragungsvorrichtung (1) nach den vorhergehenden Anspruch, wobei der freie Abstand mindestens zehnmal so groß ist wie der größte typische Partikeldurchmesser und/oder wobei der freie Abstand größer als 1/100 eines Durchmessers des Drehkörpers beträgt und insbesondere kleiner als 1/20 des Durchmessers des Drehkörpers beträgt und/oder wobei der freie Abstand größer als 70 µm beträgt und insbesondere kleiner als 300 µm beträgt und/oder wobei der freie Abstand größer als 1/500 des Außendurchmessers der inneren Komponente (2) und/oder größer als 1/500 des Innendurchmessers der äußeren Komponente (3) beträgt.

6. Magnetorheologische Übertragungsvorrichtung (1) nach wenigstens einem der vorhergehenden Ansprüche, wobei das Magnetfeld (8) durch den Drehkörper (11) und quer zu der Relativbewegung (17, 18) der Komponenten (2, 3) zueinander und von einer Komponente (2) zu der anderen Komponente (3) durch den Drehkörper (11) verläuft.

7. Magnetorheologische Übertragungsvorrichtung (1) nach wenigstens einem der vorhergehenden Ansprüche, wobei die Magnetfelderzeugungseinrichtung (7) wenigstens einen Dauermagneten (25) und wenigstens eine Spule (26) umfasst und wobei insbesondere die Magnetisierung des Dauermagneten (25) durch wenigstens einen magnetischen Impuls einer elektrischen Spule (26) dauerhaft veränderbar ist.

8. Magnetorheologische Übertragungsvorrichtung (1) nach wenigstens einem der vorhergehenden Ansprüche, welche als Bremse (40), Bedienknopf, Steuerknopf, Schwingungsdämpfer oder Kupplung (50) ausgebildet ist.

9. Magnetorheologische Übertragungsvorrichtung (1) nach wenigstens einem der vorhergehenden Ansprüche, wobei die Komponenten und/oder der Drehkörper (11) zumindest teilweise dem Magnetfeld entsprechend leitfähig und insbesondere ferromagnetisch mit einer Permeabilitätszahl größer 500 sind.

10. Magnetorheologische Übertragungsvorrichtung (1) nach wenigstens einem der vorhergehenden Ansprüche, wobei sich die Feldstärke im spitzwinkligen Bereich zwischen dem Drehkörper (11) und wenigstens einer der Komponenten (2, 3) zum Bereich mit dem geringsten Abstand hin erhöht.

11. Magnetorheologische Übertragungsvorrichtung (1) nach wenigstens einem der vorhergehenden Ansprüche, wobei an wenigstens einer der Komponenten (2, 3) wenigstens eine Erhebung angeordnet ist, die auf die andere Komponente zuragt.

12. Magnetorheologische Übertragungsvorrichtung (1) nach wenigstens einem der vorhergehenden Ansprüche, wobei der Drehkörper (11) Teil der ersten oder der zweiten Komponente (2, 3) ist und/oder wobei wenigstens ein Drehkörper (11) rund, elliptisch, zylindrisch oder eiförmig oder dergleichen ausgebildet ist und/oder wobei die Oberfläche des Drehkörpers (11) unförmig ist und/oder wobei ein haptisches Raster vorliegt.

13. Magnetorhheologische Übertragungsvorrichtung (1) nach wenigstens einem der vorhergehenden Ansprüche, wobei die Feldstärke abhängig von dem jeweiligen Abstand zwischen Drehkörper (11) und Komponenten einen starken Gradienten aufweist.

14. Magnetorheologische Übertragungsvorrichtung (1) nach wenigstens einem der vorhergehenden Ansprüche, wobei an wenigstens einer der Komponenten (2, 3) wenigstens eine Felderzeugungseinrichtung vorgesehen ist, wobei mehrere Felderzeugungseinrichtungen über dem Umfang verteilt vorgesehen sein können.

15. Verfahren zum Beeinflussen der Kopplungsintensität wenigstens zweier koppelbarer Komponenten (2, 3) mit einer magnetorheologischen Übertragungsvorrichtung (1), wobei die Kopplungsintensität in wenigstens einem Kanal (5) beeinflusst wird, der ein durch ein Magnetfeld beeinflussbares magnetorheologisches Medium (6) mit magnetisch polarisierbaren Partikeln (19) enthält, und wobei mit wenigstens einer Magnetfelderzeugungseinrichtung (7) wenigstens ein Magnetfeld (8) in dem Kanal (5) erzeugt wird, um mit dem Magnetfeld (8) das magnetorheologische Medium (6) in dem Kanal (5) zu beeinflussen, **dadurch gekennzeichnet, dass** in dem Kanal (5) wenigstens ein Drehkörper (9) vorgesehen ist, und dass ein freier Abstand (9) zwischen dem Drehkörper (11) und wenigstens einer der Komponenten (2, 3) größer ist als der doppelte Durchmesser des größten magnetisch polarisierbaren Partikels (19) in dem magnetorheologischen Medium (6), und dass zwischen dem Drehkörper (11) und wenigstens einer der Komponenten (2, 3) wenigstens ein spitzwinkliger und das magnetorheologische Medium (6) enthaltender Bereich (10) vorgesehen ist, der mit dem Magnetfeld (8) der Magnetfelderzeugungseinrichtung (7) beaufschlagt wird, um die Partikel (19) wahlweise zu verketten und/oder mit dem Drehkörper (11) zu verkeilen oder freizugeben.

## Claims

1. Magnetorheological transmission apparatus (1) comprising at least two components (2, 3) configured to be coupled, the coupling intensity of which can be influenced wherein at least one duct is provided for influencing the coupling intensity, the duct (5) comprising a magnetorheological medium (6) that can be influenced by a magnetic field and comprises magnetically polarizing particles (19), and wherein at least one magnetic field generating device (7) is provided for generating at least one magnetic field (8) in the duct (5) to influence the magnetorheological medium (6) in the duct (5) by means of the magnetic field (8), and wherein at least one rotary body (11) is provided in the duct (5),
**characterized in that** a clear distance (9) between the rotary body and at least one of the components (2, 3) is at least ten times the size of a typical medium diameter of the magnetically polarizing particles (19) in the magnetorheological medium (6), and that between the rotary body (11) and at least one of the components (2, 3) at least one section (10) is provided that shows an acute angle and contains the magnetorheological medium (6) and on which the magnetic field (8) of the magnetic field generating device (7) can be applied to selectively link, and/or wedge with the rotary body (11), or release, the particles (19).

2. The magnetorheological transmission apparatus (1) according to claim 1 wherein the acute-angled section (10) between the rotary body (11) and one of the components (2, 3) tapers down relative to the rotary body (11) in the direction of the relative motion (17, 18) of the component (2, 3).

3. The magnetorheological transmission apparatus (1) according to at least one of the preceding claims wherein the rotary body (11) is configured as a separate part (4) between the first and second components (2, 3).

4. The magnetorheological transmission apparatus (1) according to at least one of the preceding claims wherein the one component (3) is the outer component (3) surrounding the other component (2) which is the inner component (2) and/or wherein the two components (2, 3) are supported opposite one another by means of at least one separate bearing (42).

5. The magnetorheological transmission apparatus (1) according to the preceding claim wherein the clear distance is at least ten times the largest typical particle diameter and/or wherein the clear distance is larger than 1/100 of a diameter of the rotary body and is in particular smaller than 1/20 of the diameter of the rotary body and/or wherein the clear distance is larger than 70 µm and in particular smaller than 300 µm and/or wherein the clear distance is larger than 1/500 of the outer diameter of the inner component (2) and/or larger than 1/500 of the inner diameter of the outer component (3).

6. The magnetorheological transmission apparatus (1) according to at least one of the preceding claims wherein the magnetic field (8) runs through the rotary body (11) and transverse to the relative motions (17, 18) between the components (2, 3) and from one of the components (2) to the other of the components (3) through the rotary body (11).

7. The magnetorheological transmission apparatus (1) according to at least one of the preceding claims wherein the magnetic field generating device (7) comprises at least one permanent magnet (25) and at least one coil (26) and wherein in particular the magnetization of the permanent magnet (25) can be permanently changed by at least one magnetic pulse of an electric coil (26).

8. The magnetorheological transmission apparatus (1) according to at least one of the preceding claims which is configured as a brake (40), operating knob, control knob, vibration damper, or clutch (50).

9. The magnetorheological transmission apparatus (1) according to at least one of the preceding claims wherein the components and/or the rotary body (11) are at least partially conductive corresponding to the magnetic field and in particular ferromagnetic, having a relative permeability of larger than 500.

10. The magnetorheological transmission apparatus (1) according to at least one of the preceding claims wherein the field intensity increases in the acute-angled section between the rotary body (11) and at least one of the components (2, 3) toward the section showing the narrowest distance.

11. The magnetorheological transmission apparatus (1) according to at least one of the preceding claims wherein at least one of the components (2, 3) shows at least one elevation disposed thereat protruding toward the other of the components.

12. The magnetorheological transmission apparatus (1) according to at least one of the preceding claims wherein the rotary body (11) is part of the first or the second component (2, 3) and/or wherein at least one rotary body (11) is configured in a round, elliptical, cylindrical or ovoid shape or the like and/or wherein the surface of the rotary body (11) is amorphous and/or wherein a haptic grid is present.

13. The magnetorheological transmission apparatus (1) according to at least one of the preceding claims wherein the field intensity shows a large gradient dependent on the given distance between the rotary body (11) and components.

14. The magnetorheological transmission apparatus (1) according to at least one of the preceding claims wherein at least one of the components (2, 3) is provided with at least one field generating device wherein multiple field generating devices may be provided distributed over the circumference.

15. Method for influencing the coupling intensity of at least two components (2, 3) provided to be coupled, by means of a magnetorheological transmission apparatus (1) wherein the coupling intensity is influenced in at least one duct (5) containing a magnetorheological medium (6) that can be influenced by a magnetic field and contains magnetically polarizing particles (19), and wherein at least one magnetic field generating device (7) generates at least one magnetic field (8) in the duct (5) for influencing by way of the magnetic field (8) the magnetorheological medium (6) in the duct (5), **characterized in that** at least one rotary body (9) is provided in the duct (5), and that a clear distance (9) between the rotary body (11) and at least one of the components (2, 3) is larger than twice the diameter of the largest magnetically polarizing particle (19) in the magnetorheological medium (6), and that between the rotary body (11) and at least one of the components (2, 3) at least one acute-angled section (10) containing the magnetorheological medium (6) is provided on which the magnetic field (8) of the magnetic field generating device (7) is applied to selectively link, and/or wedge with the rotary body (11), or release, the particles (19).

## Revendications

1. Dispositif de transmission magnétorhéologique (1) comprenant au moins deux composants (2, 3) aptes à être couplés dont l'intensité de couplage peut être influencée, au moins un canal étant prévu pour influencer l'intensité de couplage, ledit canal (5) contenant un milieu magnétorhéologique (6) à particules (19) magnétiquement polarisables, qui peut être influencé par un champ magnétique, et au moins un dispositif de génération de champ magnétique (7) étant prévu pour générer au moins un champ magnétique (8) dans ledit canal (5) afin d'influencer par le champ magnétique (8) ledit milieu magnétorhéologique (6) dans le canal (5), et au moins un corps de rotation (11) étant prévu dans le canal (5),
**caractérisé par le fait qu'**une distance libre (9) entre ledit corps de rotation et au moins l'un des composants (2, 3) est égale à au moins dix fois un diamètre moyen typique des particules (19) magnétiquement polarisables dans le milieu magnétorhéologique (6), et qu'au moins une zone (10) à angle aigu et contenant ledit milieu magnétorhéologique (6) est prévue entre ledit corps de rotation (11) et au moins l'un des composants (2, 3), qui peut être soumise au champ magnétique (8) du dispositif de génération de champ magnétique (7) pour, au choix, enchaîner lesdites particules (19) et/ou caler celles-ci avec ledit corps de rotation (11) ou libérer celles-ci.

2. Dispositif de transmission magnétorhéologique (1) selon la revendication 1, dans lequel ladite zone à angle aigu (10) entre ledit corps de rotation (11) et l'un des composants (2, 3) se rétrécit en direction du mouvement relatif (17, 18) du composant (2, 3) par rapport au corps de rotation (11).

3. Dispositif de transmission magnétorhéologique (1) selon l'une au moins des revendications précédentes, dans lequel ledit corps de rotation (11) est réalisé comme pièce séparée (4) entre lesdits premier et deuxième composants (2, 3).

4. Dispositif de transmission magnétorhéologique (1) selon l'une au moins des revendications précédentes, dans lequel ledit un composant (3) entoure, en tant que composant extérieur (3), ledit autre composant (2) en tant que composant intérieur (2), et/ou dans lequel les deux composants (2, 3) sont logés en regard l'un de l'autre par au moins un palier (42) séparé.

5. Dispositif de transmission magnétorhéologique (1) selon la revendication précédente, dans lequel ladite distance libre est égale à au moins dix fois le plus grand diamètre de particule typique, et/ou dans lequel la distance libre est supérieure à 1/100ème d'un diamètre du corps de rotation et, en particulier, inférieure à 1/20ème du diamètre du corps de rotation, et/ou dans lequel la distance libre est supérieure à 70 µm et, en particulier, inférieure à 300 µm, et/ou dans lequel la distance libre est supérieure à 1/500ème du diamètre extérieur du composant intérieur (2) et/ou supérieure à 1/500ème du diamètre intérieur du composant extérieur (3).

6. Dispositif de transmission magnétorhéologique (1) selon l'une au moins des revendications précédentes, dans lequel le champ magnétique (8) passe à travers le corps de rotation (11) et transversalement au mouvement relatif (17, 18) des composants (2, 3) l'un par rapport à l'autre ainsi que d'un composant (2) à l'autre composant (3) à travers ledit corps de rotation (11).

7. Dispositif de transmission magnétorhéologique (1) selon l'une au moins des revendications précédentes, dans lequel ledit dispositif de génération de champ magnétique (7) comprend au moins un aimant permanent (25) et au moins une bobine (26), et dans lequel en particulier l'aimantation de l'aimant permanent (25) peut être modifiée de manière durable par au moins une impulsion magnétique d'une bobine électrique (26).

8. Dispositif de transmission magnétorhéologique (1) selon l'une au moins des revendications précédentes, qui est réalisé en tant que frein (40), bouton de manoeuvre, bouton de commande, amortisseur de vibrations ou embrayage (50).

9. Dispositif de transmission magnétorhéologique (1) selon l'une au moins des revendications précédentes, dans lequel les composants et/ou le corps de rotation (11) sont, au moins en partie, conducteurs conformément au champ magnétique et, en particulier, ferromagnétiques avec une perméabilité relative supérieure à 500.

10. Dispositif de transmission magnétorhéologique (1) selon l'une au moins des revendications précédentes, dans lequel l'intensité de champ dans la zone à angle aigu entre ledit corps de rotation (11) et au moins l'un des composants (2, 3) augmente vers la zone ayant la plus faible distance.

11. Dispositif de transmission magnétorhéologique (1) selon l'une au moins des revendications précédentes, dans lequel sur au moins l'un des composants (2, 3) est disposée au moins une éminence qui fait saillie vers l'autre composant.

12. Dispositif de transmission magnétorhéologique (1) selon l'une au moins des revendications précédentes, dans lequel ledit corps de rotation (11) fait partie du premier ou du deuxième composant (2, 3) et/ou dans lequel au moins un corps de rotation (11) est réalisé de manière à être rond, elliptique, cylindrique ou ovale ou similaire, et/ou dans lequel la surface du corps de rotation (11) est informe et/ou dans lequel il y a une trame haptique.

13. Dispositif de transmission magnétorhéologique (1) selon l'une au moins des revendications précédentes, dans lequel l'intensité de champ présente, en fonction de la distance respective séparant le corps de rotation (11) et les composants, un fort gradient.

14. Dispositif de transmission magnétorhéologique (1) selon l'une au moins des revendications précédentes, dans lequel sur au moins l'un des composants (2, 3) est prévu au moins un dispositif de génération de champ, plusieurs dispositifs de génération de champ pouvant être prévus répartis sur la circonférence.

15. Procédé pour influencer l'intensité de couplage d'au moins deux composants (2, 3) aptes à être couplés, par un dispositif de transmission magnétorhéologique (1), dans lequel l'intensité de couplage est influencée dans au moins un canal (5) qui contient un milieu magnétorhéologique (6) à particules (19) magnétiquement polarisables, qui peut être influencé par un champ magnétique, et dans lequel au moins un champ magnétique (8) est généré dans ledit canal (5) par au moins un dispositif de génération de champ magnétique (7) afin d'influencer par le champ magnétique (8) ledit milieu magnétorhéologique (6) dans le canal (5),
**caractérisé par le fait qu'**au moins un corps de rotation (11) est prévu dans le canal (5) et qu'une distance libre (9) entre ledit corps de rotation (11) et au moins l'un des composants (2, 3) est supérieure à deux fois le diamètre de la plus grande particule (19) magnétiquement polarisable dans le milieu magnétorhéologique (6), et qu'au moins une zone (10) à angle aigu et contenant ledit milieu magnétorhéologique (6) est prévue entre ledit corps de rotation (11) et au moins l'un des composants (2, 3), qui est soumise au champ magnétique (8) du dispositif de génération de champ magnétique (7) pour, au choix, enchaîner lesdites particules (19) et/ou caler celles-ci avec ledit corps de rotation (11) ou libérer celles-ci.
